(19) Europäisches Patentamt
European Patent Office
Office européen des brevets

(11) EP 0 950 661 B1

(12) **EUROPEAN PATENT SPECIFICATION**

(45) Date of publication and mention of the grant of the patent: **12.11.2003 Bulletin 2003/46**

(51) Int Cl.[7]: **C07D 513/04**, C07D 471/04, C07D 491/04, A61K 31/40

(21) Application number: **99302969.3**

(22) Date of filing: **16.04.1999**

(54) **Substituted tricyclics useful in sPLA2 induced diseases**

Substituierte tricyclische Verbindungen und deren Verwendung zur Behandlung von sPLA2-bedingten Erkrankungen

Composés tricycliques substitués pour le traitement des maladies induites par sPLA2

(84) Designated Contracting States:
**AT BE CH CY DE DK ES FI FR GB GR IE IT LI LU NL PT SE**
Designated Extension States:
**AL LT LV RO SI**

(30) Priority: **17.04.1998 US 62165**

(43) Date of publication of application:
**20.10.1999 Bulletin 1999/42**

(60) Divisional application:
**01203116.7 / 1 156 050**

(73) Proprietor: **ELI LILLY AND COMPANY**
**Indianapolis, Indiana 46285 (US)**

(72) Inventors:
- **Bach, Nicholas James**
  **Indianapolis, Indiana 46217 (US)**
- **Bastian, Jolie Anne**
  **Beech Grove, Indiana 46107 (US)**
- **Beight, Douglas Wade**
  **Frankfort, Indiana 46041 (US)**
- **Kinnick, Michael Dean**
  **Indianapolis, Indiana 46217 (US)**
- **Martinelli, Michael John**
  **Zionsville, Indiana 46077 (US)**
- **Mihelich, Edward David**
  **Carmel, Indiana 46032 (US)**
- **Morin, John Michael Jr.**
  **Brownsburg, Indiana 46112 (US)**
- **Sall, Daniel Jon**
  **Greenwood, Indiana 46142 (US)**
- **Sawyer, Jason Scott**
  **Indianapolis, Indiana 46220 (US)**
- **Smith, Edward C.R.**
  **Fishers, Indiana 46038 (US)**
- **Suarez, Tulio**
  **Greenwood, Indiana 46143 (US)**
- **Wang, Qiuping**
  **Fishers, Indiana 46038 (US)**
- **Wilson, Thomas Michael**
  **Speedway, Indiana 46224 (US)**

(74) Representative: **Vaughan, Jennifer Ann et al**
**Lilly Research Centre,**
**Erl Wood Manor**
**Windlesham, Surrey GU20 6PH (GB)**

(56) References cited:
**EP-A- 0 779 271**
**EP-A- 0 839 806**
**WO-A-96/03383**
**WO-A-98/18464**
**WO-A-99/16453**
**WO-A-99/25340**
**US-A- 3 979 391**

- **GARRATT P J ET AL: "MAPPING THE MELATONIN RECEPTOR. 2. SYNTHESIS AND BIOLOGICAL ACTIVITY OF INDOLE DERIVED MELATONIN ANALOGUES WITH RESTRICTED CONFORMATIONS OF THE C-3 AMIDOETHANE SIDE CHAIN" BIOORGANIC & MEDICINAL CHEMISTRY LETTERS, vol. 4, no. 13, 1 January 1994, pages 1559-1564, XP002054522**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 950 661 B1

## Description

This invention relates to novel substituted tricyclic organic compounds useful for inhibiting $sPLA_2$ mediated release of fatty acids for conditions such as septic shock.

The structure and physical properties of human non-pancreatic secretory phospholipase $A_2$ (hereinafter called, "$sPLA_2$") has been thoroughly described in two articles, namely, "Cloning and Recombinant Expression of Phospholipase $A_2$ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; The Journal of Biological Chemistry, Vol. 264, No. 10, Issue of April 5, pp. 5335-5338, 1989; and "Structure and Properties of a Human Non-pancreatic Phospholipase $A_2$" by Kramer, Ruth M.; Hession, Catherine; Johansen, Berit; Hayes, Gretchen; McGray, Paula; Chow, E. Pingchang; Tizard, Richard; and Pepinsky, R. Blake; The Journal of Biological Chemistry, Vol. 264, No. 10, Issue of April 5, pp. 5768-5775, 1989; the disclosures of which are incorporated herein by reference.

It is believed that $sPLA_2$ is a rate limiting enzyme in the arachidonic acid cascade which hydrolyzes membrane phospholipids. Thus, it is important to develop compounds which inhibit $sPLA_2$ mediated release of fatty acids (e.g., arachidonic acid). Such compounds would be of value in general treatment of conditions induced and/or maintained by overproduction of $sPLA_2$ such as septic shock, adult respiratory distress syndrome, pancreatitis, trauma-induced shock, bronchial asthma, allergic rhinitis, rheumatoid arthritis, etc.

It is desirable to develop new compounds and treatments for $sPLA_2$ induced diseases.

Alexander, et al., United States Patent Nos. 3,939,177 and 3,979,391, disclose 1,2,3,4-tetrahydrocarbazoles useful as antibacterial agents.

This invention provides tricyclic compounds as defined in Claim 1 below.

These substituted tricyclics are effective in inhibiting human $sPLA_2$ mediated release of fatty acids.

This invention is also a pharmaceutical formulation comprising a compound of Claim 1 in association with one or more pharmaceutically acceptable diluents, carriers and excipients.

This invention is also the use of a compound of Claim 1 for the manufacture of a medicament for inhibiting $sPLA_2$ in a mammal.

According to a further aspect of the present invention, there is provided the use of a compound of Claim 1 for the manufacture of a medicament for selectively inhibiting $sPLA_2$ in a mammal.

This invention also provides the use of a compound of Claim 1 for the manufacture of a medicament for alleviating the pathological effects of sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis and related diseases.

Other objects, features and advantages of the present invention will become apparent from the subsequent description and the appended claims.

Definitions:

The salts of the above tricyclics are an additional aspect of the invention. In those instances where the compounds of the invention possess acidic functional groups various salts may be formed which are more water soluble and physiologically suitable than the parent compound. Representative pharmaceutically acceptable salts include but are not limited to the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, *et al.*, "Pharmaceutical Salts," J. Phar. Sci., 66: 1-19 (1977)).

Compounds of the invention may have chiral centers and exist in optically active forms. R- and S-isomers

and racemic mixtures are contemplated by this invention. A particular stereoisomer may be prepared by known methods using stereospecific reactions with starting materials containing asymmetric centers already resolved or, alternatively, by subsequent resolution of mixtures of stereoisomers using known methods.

The term "acid protecting group" is used herein as it is frequently used in synthetic organic chemistry, to refer to a group which will prevent an acid group from participating in a reaction carried out on some other functional group in the molecule, but which can be removed when it is desired to do so. Such groups are discussed by T. W. Greene in chapter 5 of Protective Groups in Organic Synthesis, John Wiley and Sons, New York, 1981, incorporated herein by reference in its entirety.

Examples of acid protecting groups include ester or amide derivatives of the acid group, such as, methyl, methoxymethyl, methyl-thiomethyl, tetrahydropyranyl, methoxyethoxymethyl, benzyloxymethyl, phenyl, aryl, ethyl, 2,2,2-trichloroethyl, 2-methylthioethyl, t-butyl, cyclopentyl, triphenylmethyl, diphenylmethyl, benzyl, trimethylsilyl, N,N-dimethyl, pyrrolidinyl, piperidinyl, or o-nitroanilide. A preferred acid-protecting group is methyl.

Example 1

Preparation of (*R,S*)-(9-Benzyl-4-carbamoyl-1-oxo-3-thia-1,2,3,4-tetrahydrocarbazol-5-yl)oxyacetate Acid

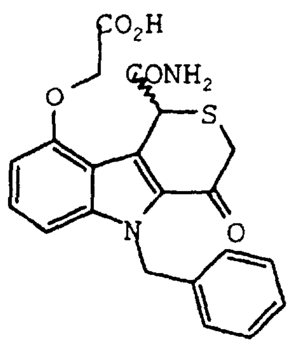

A. 1-Benzyl-4-methoxyindole

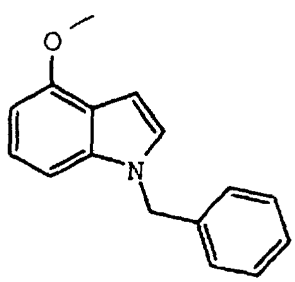

NaH (7.7 g, 191.7 mmol) was added portionwise to a 0 °C solution of 4-methoxyindole (21.7 g, 147 mmol) in 750 mL of anhydrous DMF. After 15 min, the slurry was treated with benzyl bromide (17.5 mL, 147 mmol). The reaction mixture was allowed to warm to ambient temperature and stir overnight. The reaction mixture was poured into 1 L of $H_2O$. The layers were separated, and the aqueous phase was extracted with EtOAc (2 X 200 mL). The combined organic layers were washed with $H_2O$ (4 x 500 mL), dried over $Na_2SO_4$, filtered and concentrated *in vacuo*. The crude residue was purified by flash chromatography ($SiO_2$; hexanes) to give 32.9 g (138.6 mmol; 94%) of the title compound as a white solid. Electrospray MS 238 (M+1);

| Elemental Analysis for $C_{16}H_{15}NO$: | | | |
|---|---|---|---|
| Calculated | C, 80.98; | H, 6.37; | N, 5.90. |
| Found | C, 81.20; | H, 6.09; | N, 5.83. |

B. Methyl (1-Benzyl-4-methoxyindol-3-yl)oxoacetate

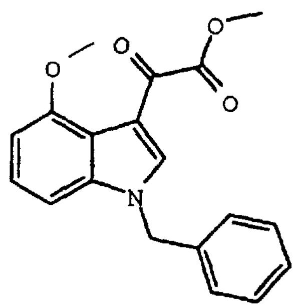

A 0 °C solution of 1-benzyl-4-methoxyindole (31.9 g, 134.4 mmol) in 500 mL of $CH_2Cl_2$ and pyridine (21.7 mL, 268.8 mmol) was treated with methyl oxalyl chloride (13.6 mL, 147.9 mmol). After 1.5 h at 0 °C, 500 mL of saturated $NaHCO_3$ solution was added. The aqueous layer was extracted with $CHCl_3$ (1 x 200 mL, 2 x 50 mL). The combined organic layers were concentrated *in vacuo* to a tan solid, which was triturated with EtOAc/hexanes to give 29.8 g (92.1 mmol; 69%) of the title compound as an off-white powder. Electrospray MS 324 (M+1);

| Elemental Analysis for $C_{19}H_{17}NO_4$: | | | |
|---|---|---|---|
| Calculated | C, 70.58; | H, 5.30; | N, 4.33. |
| Found | C, 70.86; | H, 5.42; | N, 4.49. |

C. Methyl (*R,S*)-(1-Benzyl-4-methoxyindol-3-yl)hydroxyacetate

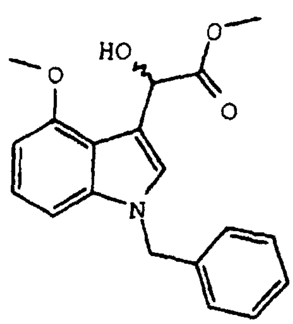

A solution of methyl (1-benzyl-4-methoxyindol-3-yl)oxoacetate (10 g, 30.9 mmol) in 300 mL of MeOH was treated with $NaBH_4$ (1.46 g, 38.6 mmol). After stirring overnight, EtOAc and $H_2O$ (20 mL each) were added. The aqueous layer was extracted with EtOAc (2 x 25 mL). The combined organic layers were dried over $Na_2SO_4$, filtered and concentrated *in vacuo*. The crude residue was recrystallized with EtOAc/hexanes to give 9.1 g (28.0 mmol; 91%) of the title compound as a white powder. FDMS 325 (M+);

| Elemental Analysis for $C_{19}H_{19}NO_4$: | | | |
|---|---|---|---|
| Calculated | C, 70.14; | H, 5.89; | N, 4.30. |
| Found | C, 70.42; | H, 5.93; | N, 4.41. |

D. (*R,S*)-[(1-Benzyl-4-methoxyindol-3-yl)(carbomethoxy)methyl]thioacetic Acid

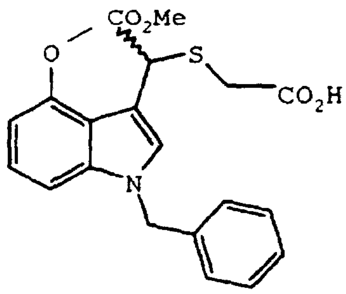

A 0 °C slurry of methyl (*R,S*)-(1-benzyl-4-methoxyindol-3-yl)hydroxyacetate (3.5 g, 10.8 mmol) and $K_2CO_3$ (2.2 g, 16.1 mmol) in 50 mL of $CH_2Cl_2$ was treated with TEA (0.075 mL, 0.54 mmol). After 15 min, MsCl (1.25 mL, 16.1 mmol) was added. After stirring for 2 h at 0 °C, mercaptoacetic acid (3 mL, 43 mmol) was added, and the reaction was heated at reflux overnight. The reaction mixture was poured into 25 mL of saturated $NaHCO_3$ solution. The aqueous layer was extracted with 25 mL of $CHCl_3$, acidified with 1 N HCl and extracted again with $CHCl_3$ (3 x 25 mL). The acidified extracts were combined, dried over $Na_2SO_4$, filtered and concentrated *in vacuo*. The oily residue was purified by flash chromatography ($SiO_2$; gradient of 0% to 2% glacial acetic acid in 1:1 EtOAc/hexanes) to give 2.58 g (6.46 mmol; 60%) of the title compound as a clear oil which solidified on standing. FDMS 399 (M+);

| Elemental Analysis for $C_{21}H_{21}NO_5S \cdot 0.2H_2O$: | | | |
|---|---|---|---|
| Calculated | C, 62.58; | H, 5.35; | N, 3.48. |
| Found | C, 62.57; | H, 5.26; | N, 3.55. |

E. Methyl (*R,S*)-(9-Benzyl-5-methoxy-1-oxo-3-thia-1,2,3,4-tetrahydrocarbazol-5-yl)carboxylate

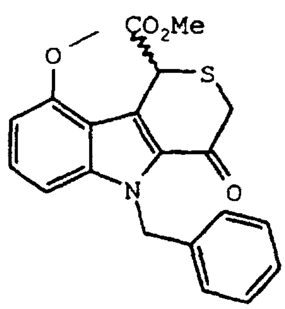

A solution of the carboxylic acid from Part D above (2.32 g, 5.81 mmol) in 50 mL of 1,2-dichloroethane was treated with oxalyl chloride (2.0 mL, 22.9 mmol) and 1 drop of DMF. The resulting mixture was allowed to stir at ambient temperature for 3 h, then it was concentrated *in vacuo*. The crude residue was purified by flash chromatography ($SiO_2$; gradient of 0% to 5% to 10% EtOAc/hexanes) to give 1.39 g (3.64 mmol; 63%) of the title compound as a pale yellow powder. FDMS 381 (M+);

| Elemental Analysis for $C_{21}H_{19}NO_4S$: | | | |
|---|---|---|---|
| Calculated | C, 66.12; | H, 5.02; | N, 3.67. |
| Found | C, 66.00; | H, 5.26; | N, 3.63. |

F. Methyl (*R,S*)-(9-Benzyl-5-methoxy-1-oxo-3-thia-1,2,3,4-tetrahydrocarbazol-5-yl)carboxamide

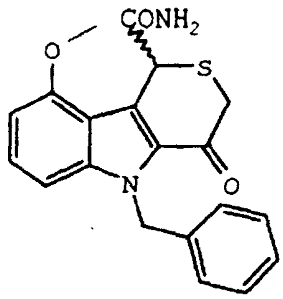

A solution of methyl (*R,S*)-(9-benzyl-5-methoxy-1-oxo-3-thia-1,2,3,4-tetrahydrocarbazol-5-yl)carboxylate (1.1 g, 2.88 mmol) in 25 mL of THF/MeOH/$H_2O$ (3:1:1) was treated with LiOH (83 mg, 3.46 mmol) and allowed to stir at ambient temperature overnight. The aqueous layer was extracted with 25 mL of $CH_2Cl_2$, acidified with 1 N HCl and extracted again with $CH_2Cl_2$ (2 x 25 mL). The acidified extracts were combined, dried over $Na_2SO_4$, filtered and concentrated *in vacuo*. The crude intermediate acid was dissolved in 20 mL of 1,2 dichloroethane and treated with $(COCl)_2$ (0.77 mL, 8.82 mmol). After 4 h, the reaction mixture was concentrated *in vacuo* and resuspended in 20 mL of 1,2 dichloroethane. Ammonia was bubbled through the solution for *ca*. 10 min, then the reaction mixture was capped and allowed to stand for 1.5 h. The crude amide was concentrated *in vacuo* and recrystallized from EtOAc/hexanes to give 780 mg (2.13 mmol; 74%) of the title compound as a light tan solid. FDMS 366 (M+);

| Elemental Analysis for $C_{20}H_{18}N_2O_4S{\cdot}0.2H_2O$: | | | |
|---|---|---|---|
| Calculated | C, 64.92; | H, 5.01; | N, 7.57. |
| Found | C, 64.95; | H, 5.04; | N, 7.78. |

G. (*R*,*S*)-(9-Benzyl-5-hydroxy-1-oxo-3-thia-1,2,3,4-tetrahydrocarbazol-5-yl)carboxamide

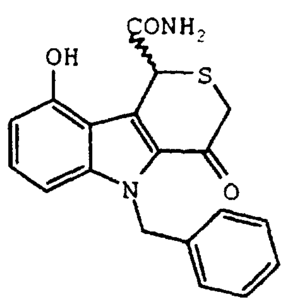

A 0 °C solution of methyl (*R,S*)-(9-benzyl-5-methoxy-1-oxo-3-thia-1,2,3,4-tetrahydrocarbazol-5-yl)carboxamide in 10 mL of 1,2 dichloroethane was treated with $BBr_3$ (2.4 mL, 24.9 mmol). After 3 h, the reaction mixture was quenched cold with MeOH and poured into 20 mL of saturated $NaHCO_3$ solution. The aqueous layer was extracted with $CHCl_3$ (4 x 50 mL). The combined organic layers were dried over $Na_2SO_4$, filtered and concentrated in vacuo. Purification of the crude residue by radial chromatography ($SiO_2$; gradient of 0% to 2% MeOH/$CHCl_3$) to give 162 mg

(0.46 mmol; 28%) of the title compound as brown foam. FDMS 352 (M+);

| Elemental Analysis for $C_{19}H_{16}N_2O_3S{\cdot}0.8H_2O$: | | | |
|---|---|---|---|
| Calculated | C, 62.21; | H, 4.84; | N, 7.64. |
| Found | C, 62.57; | H, 4.50; | N, 7.27. |

H. Ethyl (*R,S*)-(9-Benzyl-4-carbamoyl-1-oxo-3-thia-1,2,3,4-tetrahydrocarbazol-5-yl)oxyacetate

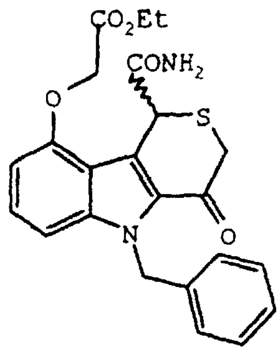

A slurry of (*R,S*)-(9-benzyl-5-hydroxy-1-oxo-3-thia-1,2,3,4-tetrahydrocarbazol-5-yl)carboxamide (145 mg, 0.411 mmol) and $Cs_2CO_3$ (400 mg, 1.23 mmol) in 5 mL of DMF was treated with ethyl bromoacetate (0.046 mL, 0.411 mmol). After stirring overnight, the reaction mixture was poured into 20 mL of $H_2O$. The aqueous layer was extracted with EtOAc (4 x 50 mL). The combined organic layers were washed with $H_2O$ (3 x 100 mL), dried over $Na_2SO_4$, filtered and concentrated *in vacuo*. Purification of the crude residue by radial chromatography ($SiO_2$; gradient of 0% to 0.5% MeOH/$CHCl_3$) afforded 120 mg (0.274 mmol; 67%) of the title compound as light tan foam. FDMS 438 (M+);

| Elemental Analysis for $C_{23}H_{22}N_2O_5S{\cdot}0{\cdot}3H_2O{\cdot}0{\cdot}4CHCl_3$: | | | |
|---|---|---|---|
| Calculated | C, 57.16; | H, 4.72; | N, 5.70. |
| Found | C, 57.18; | H, 4.61; | N, 5.68. |

I. (*R,S*)-(9-Benzyl-4-carbamoyl-1-oxo-3-thia-1,2,3,4-tetrahydrocarbazol-5-yl)oxyacetic Acid

A solution of ethyl (*R,S*)-(9-benzyl-4-carbamoyl-1-oxo-3-thia-1,2, 3,4-tetrahydrocarbazol-5-yl)oxyacetate (20 mg, 0.0456 mmol) in 0.5 mL of THF/MeOH/$H_2O$ (3:1:1) was treated with LiOH (1.3 mg, 0.0547 mmol). The solution quickly turned clear orange, and after 45 min, the aqueous layer was extracted with 10 mL of $CHCl_3$, acidified with 1 N HCl and extracted again with $CHCl_3$ (3 x 20 mL). The acidified extracts were combined, dried over $Na_2SO_4$, filtered and concentrated *in vacuo* to an orange solid. The crude acid was purified on a pipet column ($SiO_2$; gradient of 0 to 2% MeOH/$CHCl_3$, trace glacial acetic acid) to afford 10 mg (0.0244 mmol; 53%) of the title compound as a light tan solid. FAB HRMS: m/e for $C_{21}H_{19}N_2O_5S$: 411.1015. Found: 411.1010 (M+1).

Example 2

Preparation of (R,S)-(9-Benzyl-4-carbamoyl-3-thia-1,2,3,4-tetrahydrocarbazol-5-yl)oxyacetic Acid

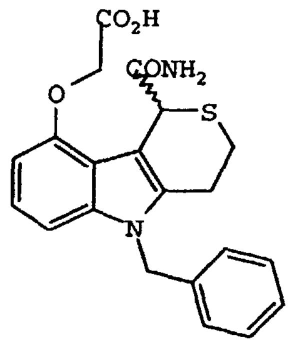

A. Ethyl (*R,S*)-(9-Benzyl-4-carbamoyl-3-thia-1,2,3,4-tetrahydrocarbazol-5-yl)oxyacetate

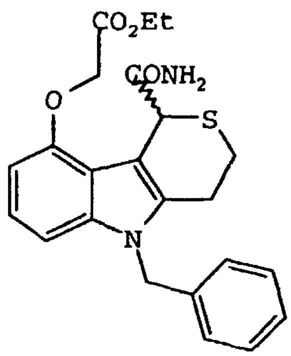

A slurry of ethyl (*R,S*)-(9-benzyl-4-carbamoyl-1-oxo-3-thia-1,2,3,4-tetrahydrocarbazol-5-yl)oxyacetate (75 mg, 0.171 mmol) in 1 mL of MeOH and 1.5 mL of THF (for solubility) was treated with $NaBH_4$ (8 mg, 0.214 mmol). After 20 min, the reaction mixture was quenched with 10 mL of $H_2O$. The layers were separated, and the aqueous layer was extracted with EtOAc (3 x 20 mL). The combined organic layers were dried over $K_2CO_3$, filtered and concentrated *in vacuo.* The crude intermediate alcohol was immediately dissolved in 2 mL of 1,2-dichloroethane. The resulting solution was treated with $Et_3SiH$ (0.19 mL, 1.2 mmol). Upon cooling to 0 °C, TFA (0.13 mL, 1.7 mmol) was added dropwise. After 1 h, the reaction mixture was poured into 25 mL of saturated aqueous $NaHCO_3$. The layers were separated, and the aqueous layer was extracted with EtOAc (3 x 25 mL). The combined organic layers were dried over $Na_2SO_4$, filtered and concentrated *in vacuo*. Purification of the crude residue by flash chromatograhy ($SiO_2$; gradient of 0% to 0.5% MeOH/$CHCl_3$) afforded 38 mg (0.0895 mmol, 52%) of the title compound as an off-white solid. FDMS 424 (M+);

| Elemental Analysis for $C_{23}H_{24}N_2O_4S{\cdot}0{\cdot}3H_2O{\cdot}0{\cdot}6CHCl_3$: | | | |
|---|---|---|---|
| Calculated | C, 56.51; | H, 5.06; | N, 5.59. |
| Found | C, 56.61; | H, 4.87; | N, 5.60. |

B. (R,S)-(9-Benzyl-4-carbamoyl-1-oxo-3-thia-1,2,3,4-tetrahydrocarbazol-5-yl)oxyacetic Acid ).

A solution of ethyl (*R,S*)-(9-benzyl-4-carbamoyl-3-thia-1,2,3,4-tetrahydrocarbazol-5-yl)oxyacetate (28 mg,

0.066 mmol) in 0.5 mL of THF/MeOH/$H_2O$ (3:1:1) was treated with LiOH (1.9 mg, 0.079 mmol). After 1 h, the aqueous layer was extracted with 10 mL of $CHCl_3$, acidified with 1 N HCl and extracted again with $CHCl_3$ (3 x 20 mL). The organics were combined, dried over $Na_2SO_4$, filtered and concentrated *in vacuo*. The crude acid was purified on a pipet column ($SiO_2$; gradient of 0 to 1% MeOH/$CHCl_3$, trace glacial acetic acid) to afford 18 mg (0.045 mmol; 69%) of the title compound as an off-white solid. FAB HRMS: m/e, calcd for $C_{21}H_{21}N_2O_4S$: 397.1222., Found: 397.1216 (m+1).

Example 3

2-(4-oxo-5-carboxamido-9-benzyl-*9H*-pyrido[3,4-*b*]indolyl)acetic acid hydrochloride

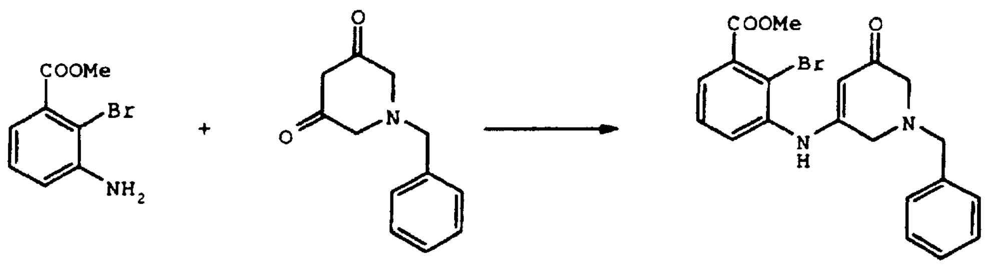

A. Preparation of N-[5-(1-benzyl-3-oxo-1,2,3,6-tetrahydropyridinyl)]-2-bromo-3-carbomethoxyaniline

To a mixture of 2-bromo-3-carbomethoxyaniline (12.0 g, 52.2 mmol) and pyridinium p-toluenesulfonate (13.8 g, 54.9 mmol) in 2:1 toluene/dioxane (300 mL) was added 1-benzyl-3,5-piperidinedione (13.0 g, 70.2 mmol, Chen, L. -C.; Yang, S.-C. *Heterocycles* **1990,** *31*, 911-916). The apparatus was fitted with a Dean-Starke trap and the mixture refluxed for 10 h. The mixture was concentrated in vacuo and the residue dissolved in chloroform. This solution was washed three times with water, once with saturated sodium chloride solution, dried (sodium sulfate), filtered, and concentrated in vacuo to provide a dark oil. Chromatography (silica gel, chloroform to 4% methanol/96% chloroform) provided 2.0 g (9%) of the title product as a foam which could be crystallized form acetonitrile: mp 156-158 °C. $^1H$ NMR ($CDCl_3$) d 7.55 (m, 2 H), 7.40 (m, 6 H), 5.55 (s, 1 H), 3.94 (s, 3 H), 3.85 (m, 2 H), 3.56 (m, 2 H), 3.30 (bs, 2 H); MS ES+ m/e 414.9 (p), 416.9 (p); IR (KBr, $cm^{-1}$) 3185, 2944, 1728, 1603, 1544, 1306.

| Elemental Analysis for $C_{20}H_{19}BrN_2O_3$: | | | |
|---|---|---|---|
| Calculated | C, 57.84; | H, 4.61; | N, 6.75. |
| Found | C, 58.13; | H, 4.49; | N, 6.91. |

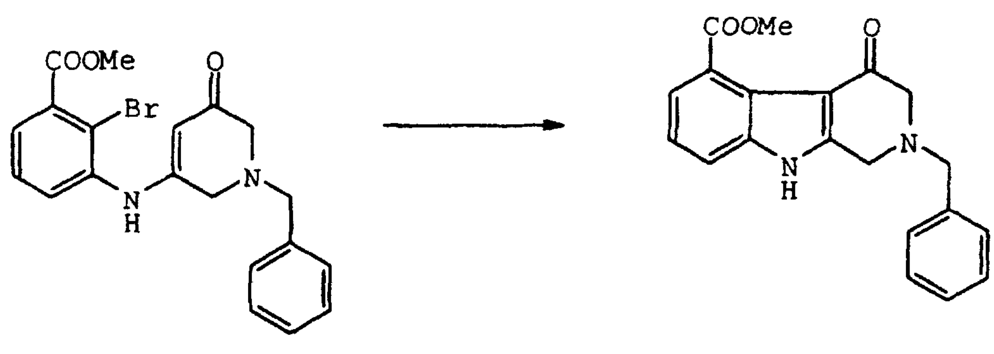

B. Preparation of 2-benzyl-4-oxo-5-carbomethoxy-1,2,3,4-tetrahydro-*9H*-pyrido[3,4-*b*]indole.

A mixture of N-[5-(1-benzyl-3-oxo-1,2,3,6-tetrahydropyridinyl)]-2-bromo-3-carbomethoxyaniline (2.07 g, 4.98 mmol), palladium(II) acetate (0.112 g, 0.499 mmol), tri-*o*-tolylphosphine (0.304 g, 0.999 mmol), triethylamine (1.3 mL,

9.3 mmol), and N,N-dimethylformamide (3 mL) in acetonitrile (12 mL) was placed in a tube and purged with argon. The tube was sealed and heated at 100 °C for 16 h. The mixture was cooled to room temperature, diluted with ethyl acetate, filtered, and the filtrate concentrated in vacuo to give a dark oil. Chromatography (silica gel, chloroform to 4% methanol/96% chloroform) provided 1.28 g (77%) of an oil which crystallized upon storing at 10 °C: recrystallized fro EtoAc/hexane mp 174-176 °C. $^1H$ NMR ($CDCl_3$) d 9.25 (bs, 1 H), 7.38 (d, J = 9 Hz, 2 H), 7.30 (m, 5 H), 7.23 (t, J = 8 Hz, 1 H), 3.97 (s, 3 H), 3.75 (s, 2 H), 3.72 (s, 2 H), 3.61 (s, 2 H); MS ES+ m/e 335 (p + 1);
IR (KBr, $cm^{-1}$) 3080, 1721, 1628, 1476, 1294, 1138.

| Elemental Analysis for $C_{20}H_{18}N_2O_3$: | | | |
|---|---|---|---|
| Calculated | C, 71.84; | H, 5.43; | N, 8.38. |
| Found | C, 72.06; | H, 5.31; | N, 8.31. |

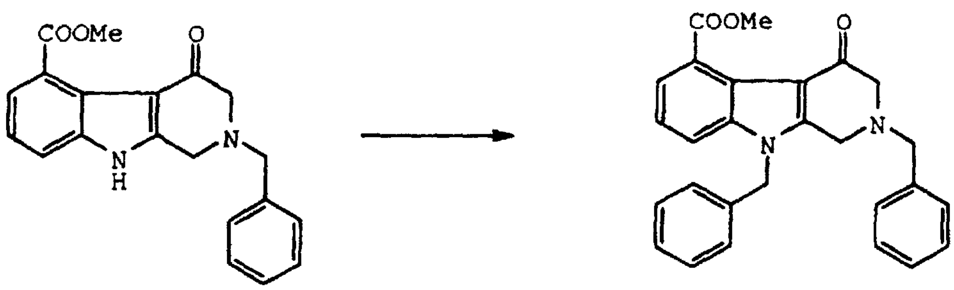

C. Preparation of 2,9-dibenzyl-4-oxo-5-carbomethoxy-1,2,3,4-tetrahydro-*9H*-pyrido[3,4-b]indole.

To a solution of 2-benzyl-4-oxo-5-carbomethoxy-1,2,3,4-tetrahydro-*9H*-pyrido[3,4-*b*]indole (0.928 g, 2.78 mmol) in dry tetrahydrofuran (5 mL) was added 60% sodium hydride in oil (111 mg). The resulting mixture was stirred at room temperature until gas evolution ceased. A solution of benzyl iodide (0.606 g, 2.78 mmol) in dry tetrahydrofuran (5 mL) was added to the reaction mixture and the resulting solution stirred at room temperature for 60 h. The mixture was diluted with methylene chloride and washed twice with saturated sodium chloride solution. The organic layer was dried (magnesium sulfate), filtered, and concentrated in vacuo. The residue was triturated with ethyl acetate resulting in a yellow precipitate (163 mg). The filtrate was concentrated in vacuo and chromatographed (silica gel, 5% methanol/ 95% methylene chloride) to provide an additional 580 mg of the title compound (743 mg total, 63%) as a crystalline solid: mp 198-199 °C. $^1H$ NMR ($CDCl_3$) d 7.43 (d, J = 7 Hz, 1 H), 7.36 (d, J = 8 Hz, 1 H), 7.25 (m, 9 H), 6.95 (m, 2 H), 5.24 (s, 2 H), 4.01 (s, 3 H), 3.78 (m, 4 H), 3.40 (bs, 2 H); MS EI+ m/e 425 (p + 1); IR (KBr, $cm^{-1}$) 1726, 1648, 1449, 1291, 1134, 1107.

| Elemental Analysis for $C_{27}H_{24}N_2O_3$: | | | |
|---|---|---|---|
| Calculated | C, 76.40; | H, 5.70; | N, 6.60. |
| Found | C, 76.11; | H, 5.45; | N, 6.54. |

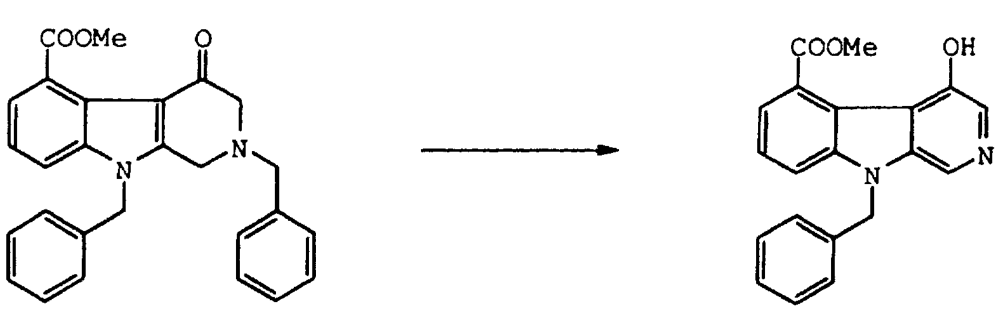

D. Preparation 4-hydroxy-5-carbomethoxy-9-benzyl-*9H*-pyrido[3,4-*b*]indole.

A mixture of of 2,9-dibenzyl-4-oxo-5-carbomethoxy-1,2,3,4-tetrahydro-*9H*-pyrido[3,4-*b*]indole (521 mg, 1.23 mmol) and 10% palladium-on-carbon (250 mg) in acetic acid (15 mL) was refluxed for 4 h. The reaction flask was cooled to room temperature and purged with nitrogen. The flask was placed under a positive pressure of hydrogen and heated at 75 °C for 16 h. The mixture was cooled to room temperature, filtered, and concentrated in vacuo to provide an orange solid. Chromatography (silica gel, 4% methanol/96% methylene chloride) provided 271 mg (60%) of the title compound as a mono-hydrated yellow powder: mp >250 °C.

$^1$H NMR ($CDCl_3$) d 8.46 (s, 1 H), 8.22 (s, 1 H), 8.09 (d, J = 8 Hz, 1 H), 7.70 (d, J = 8 Hz, 1 H), 7.56 (t, J = 8 Hz, 1 H), 7.23 (m, 3 H), 7.08 (m, 2 H), 5.60 (s, 2 H), 4.11 (s, 3 H); MS ES+ m/e 333 (p + 1).

| Elemental Analysis for $C_{20}H_{16}N_2O_3 \cdot H_2O$: | | | |
|---|---|---|---|
| Calculated | C, 68.60; | H, 4.98; | N, 7.91. |
| Found | C, 68.56; | H, 5.18; | N, 8.00. |

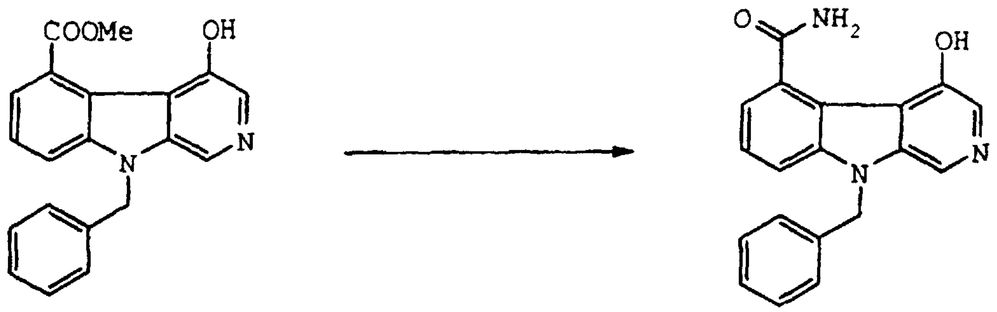

E. Preparation of 4-hydroxy-5-carboxamido-9-benzyl-*9H*-pyrido[3,4-*b*]indole.

4-Hydroxy-5-carbomethoxy-9-benzyl-*9H*-pyrido[3,4-*b*]indole (200 mg, 0.618 mmol) was dissolved in a solution of 2M methanolic ammonia (10 mL) and placed in an open tube. The solution was saturated with gaseous ammonia for 10 min. The tube was sealed and heated at 60-65 °C for 8 h. The reaction mixture was cooled to room temperature and the resulting precipitate was collected in vacuo to provide 0.12 g (61%) of the title compound as a yellow solid: mp >250 °C. $^1$H NMR (DMSO-$d_6$) d 10.99 (s, 1 H, -OH), 8.99 (bs, 1 H, -NH), 8.59 (s, 1 H), 8.55 (bs, 1 H, -NH), 7.96 (d, J = 7 Hz, 1 H), 7.94 (s, 1 H), 7.64 (t, J = 8 Hz, 1 H), 7.57 (d, J = 7 Hz, 1 H), 7.22 (m, 3 H), 7.12 (d, J = 7 Hz, 2 H), 5.80 (s, 2 H); MS ES+ m/e 318 (p + 1).

| Elemental Analysis for $C_{19}H_{15}N_3O_2$: | | | |
|---|---|---|---|
| Calculated | C, 71.91; | H, 4.76; | N, 13.24. |
| Found | C, 72.20; | H, 4.57; | N, 13.48. |

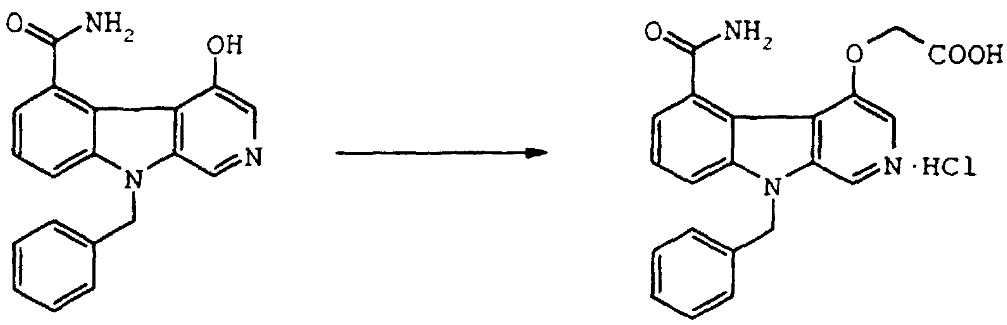

F. Preparation of 2-(4-oxo-5-carboxamido-9-benzyl-*9H*-pyrido[3,4-*b*]indolyl)acetic acid hydrochloride.

A mixture of 4-hydroxy-5-carboxamido-9-benzyl-*9H*-pyrido[3,4-*b*]indole (57 mg, 0.18 mmol), methyl bromoacetate (51 mL, 0.54 mmol), and cesium carbonate (114 mg, 0.349 mmol) in N,N-dimethylformamide (2 mL) was stirred at room temperature for 45 min. The mixture was treated with a minimum of water and methanol and concentrated in vacuo. The residue was dissolved in 1M aqueous lithium hydroxide (0.5 mL) and stirred at room temperature for 1 h. The mixture was concentrated in vacuo. The residue was dissolved in dilute aqueous hydrochloric acid and purified via reverse-phase HPLC, followed by lyophilization, to provide 28.5 mg (38%) of the title product. $^1$H NMR (DMSO-$d_6$) d 12.85 (bs, 1 H), 9.41 (s, 1 H), 9.11 (s, 1 H), 8.66 (s, 1 H), 8.30 (s, 1 H), 8.10 (d, J = 8 Hz, 1 H), 7.85 (t, J = 8 Hz, 1 H), 7.76 (d, J = 7 Hz, 1 H), 7.27 (m, 3 H), 7.19 (m, 2 H), 5.88 (s, 2 H), 5.37 (s, 2 H); MS ES+ m/e 375 (p + 1).

| Elemental Analysis for $C_{21}H_{17}N_3O_4 \cdot HCl \cdot 0.5H_2O$: | | | |
|---|---|---|---|
| Calculated | C, 60.58; | H, 4.47; | N, 10.09. |
| Found | C, 60.39; | H, 4.35; | N, 9.69. |

Example 4

Preparation of [N-benzyl-1-carbamoyl-1-aza-1,2,3,4-tetrahydrocarbazol-8-yl]oxyacetic acid

A. Preparation of methyl N-benzyl-4-methoxyindole-2-carboxylate

6.15 g of methyl 4-methoxy indole-2-carboxylate were dissolved in 30 ml of dimethylformamide, added to a slurry of 12 g of cesium carbonate in 20 ml of dimethyl formamide and warmed to 45-50 °C for 1 hour. After cooling, benzyl bromide was added in the same solvent and stirred over night at r.t. The work up was done by adding ice-water and extracting twice with ether. The ether layer was washed with water, brine, dried over magnesium sulfate , filtered and concentrated to dryness. 8.6 g (97%).
Mass Spec.: $M^+$+1 (296) mp. 104-5°C

B. Preparation of N-benzyl-2-hydroxymethyl-4-methoxyindole

To a slurry of 0.31g of lithium aluminum hydride (8.2 mmol) in 25 ml of ether at 0-10 °C was added the methyl N-benzyl-4-methoxy indole-2-carboxylate (2.95g) dissolved in 10 ml of the same solvent. The mixture was stirred at r. t. for 2 hours, quenched under the standard Fieser and Fieser procedure, filtered through a pad of celite and concentrated to dryness to give 2.8 g of alcohol. Mass spec.: $M^+$+1 (268) mp.142-3°C

C. Preparation of N-benzyl-4-methoxyindole-2-carboxaldehyde

A mixture of 3.2 g of N-benzyl-2-hydroxymethyl-4-methoxyindole (12 mmol) and 15 g of manganese dioxide (172 mmol) in 50 cc of dry dichloromethane was heated at reflux for 6 hours, cooled down to r.t. and filtered through celite. Concentration to dryness afford 3.6 g of a yellow solid. mp.130-31°C

D. Preparation of methyl N-benzyl-4-methoxyindole-2-propionate

3.1 g (11.7 mmol) of N-benzyl-4-methoxyindole-2-carboxaldehyde were combined in 20 ml of pyridine with 3.65 g (35.1 mmol) of malonic acid and 0.4 g of piperidine; the mixture was heated at 100 °C for 2 hours, concentrated under vacuum to one third of volume and acidified with 1N HCl . The solid was filtered off, washed with water and vacuum dried to give 3.0 g of product.(85%). Mass Spec.:$M^+$+1(308) mp.208-10°C. This material was dissolved in 30 ml of methanol and 1 ml of sulfuric acid, heated to reflux for 2 hours, cooled to r.t. and concentrated to a small volume. The resultant solid was isolated by filtration. This material was hydrogenated in methanol-tetrahydrofuran with 5% Pd on carbon to afford the title compound (2.5g) in 66% yield overall. Mass Spec.: $M^+$+1(324) mp.195-6°C

E. Preparation of N-benzyl-1-aza-(3,4-dihydro)-8-methoxycarbazol-2-one

2.5g of methyl N-benzyl-4-methoxyindole-2-propionate (7.7 mmol) were dissolved in 25 ml of ether and 2 equivalents (5.86g ) of bis(2,2,2-trichloroethyl)azodicarboxylate were added portionwise over half hour, stirred at r.t. over night, filtered and concentrated to dryness. This compound was dissolved in a small amount of ether and filtered to give 3.2 g of a green solid. 1 g of this complex was reduced in 5 ml of acetic acid with 1 g of activated Zn. The

temperature was kept at 10°C for 1 hour, allowed to warmed up to r.t. and stirred overnight. Water was added and basified with 1N sodium hydroxide. Extraction with tetrahydrofuran and ethyl acetate, washing, drying and concentration gave a brown oil that crystallized from isopropyl alcohol.300mg of crude and 130 mg after crystallization. Mass Spec.: $M^{+}$+1(307) mp.206-8°C.

F. Preparation of N-benzyl-1-carbamoyl-1-aza-8-methoxy-1,2,3,4-tetrahydrocarbazole

500 mg of N-benzyl-1-aza-(3,4-dihydro)-8-methoxycarbazol-2-one in tetrahydrofuran were treated with 82 mg of lithium aluminum hydride at r.t. then warmed to 50°C. Work up was done according to the Fieser and Fieser procedure, (The Agents for Organic Synthesis, Fieser, L. et al., John Wiley and Sons, NY 1967, p. 583) filtered through celite and concentrated to dryness. 420 mg of crude product. This product without further purification was treated with trimethylsilyl isocyanate in tetrahydrofuran for two hours and then concentrated to dryness. Ether was added and the amorphous solid isolated by filtration. 360 mg. Mass Spec.: $M^{+}$+1(336)

G. Preparation of [N-benzyl-1-carbamoyl-1-aza-1,2,3,4-tetrahydrocarbazol-8-yl]oxyacetic acid methyl ester.

300 mg of N-benzyl-l-carbamoyl-1-aza-8-methoxy-1,2,3,4-tetrahydrocarbazole were dissolved in 10 ml of dichloromethane and cooled down to -20°C. 10 ml of a 1M solution of Boron tribromide in the same solvent were added dropwise. It was stirred at r.t. for three hours and poured over 1 N HCl-ice. This material was extracted in ethyl acetate, washed with water, brine, dried over magnesium sulfate, filtered and concentrated to dryness to give 190 mg. This material was dissolved in 5 ml of dimethylformamide and a slight excess of cesium carbonate was added. After warming to 35 °C for 10 minutes the methyl bromoacetate added and stirred at r.t. over night. Water was added, extracted with ethyl acetate, washed ,dried over magnesium sulfate, filtered, and concentrated to dryness. Flash purification using 3:1 chloroform-ethyl acetate afforded 45 mg of product. Mass Spec.: $M^{+}$+1(394) NMR($CDCl_3$) 7.3 (m, 5 H), 7.0 (m,1 H), 6.95 (d, 1H) 6.4(d,1H) 5.25 (s,2H) 5.2 (b,2H) 4.8 (s,2H), 3.8 (s,3H), 2.75 (b,2H), 2.1 (b,2H), 1.25 (2,2H).

H. Preparation of [N-benzyl-1-carbamoyl-1-aza-1,2,3,4-tetrahydrocarbazol-8-yl]oxyacetic acid.

15 mg of [N-benzyl-1-carbamoyl-1-aza-1,2,3,4-tetrahydrocarbazol-8-yl]oxyacetic acid methyl ester were dissolved in 10ml of 7:1 tetrahydrofuran:methanol and 0.5 ml of 1N sodium hydroxide was added. After stirring at r.t. overnight, the solvents were stripped off, the residue acidified with 1N HCl and the solid filtered. This was washed with water and vacuum dried. Mass Spec.: $M^{+}$+1(380)

Example 5

Preparation of 4-methoxy-6-methoxycarbonyl-10-phenylmethyl-6,7,8,9-tetrahydropyrido[1,2-a]indole

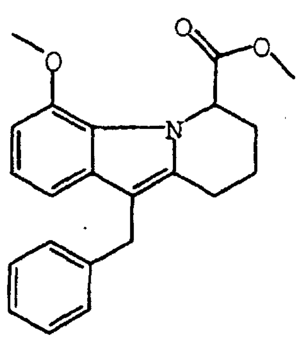

A. Preparation of 3-phenylmethyl-7-methoxyindole.

A mixture of 15gm (0.086mol) of 2-methoxyphenylhydrazine hydrochloride and 12mL (0.09mol) of 3-phenylpropionaldehyde in 300mL of toluene was refluxed for 1.5 hours with azeotropic removal of water. The suspension was cooled, evaporated *in vacuo* and the residue dissolved in 500mL of dichloromethane and stirred with 9mL (0.09mol) of phosphorous trichloride for 18 hours. The solution was poured into ice-water, stirred well, and made basic with

sodium bicarbonate. The organic phase was washed with saturated sodium chloride, dried over sodium sulfate, and evaporated *in vacuo.* The residue was chromatographed on silica gel eluting with a gradient hexane/5-15% ethyl ether to give product, 8.0gm, 40%, as a viscous oil. $^1H$ NMR ($CDCl_3$) δ: 3.95 (s, 3H), 4.10 (s, 2H), 6.65 (d, 1H), 6.90 (s, 1H), 7.00 (t, 1H), 7.10 (d, 1H), 7.20 (m, 1H), 7.30 (m, 4H), 8.20 (br s, 1H)

B. Preparation of methyl 2-[3-phenylmethyl-7-methoxyindol-1-yl]-5-chloropentanoate.

A solution of 2.7gm (llmmol) of the product from Part A in 75mL of dimethylsulfoxide and a few mL's of tetrahydrofuran was treated in portions with 480mg of sodium hydride (60% in mineral oil, 12mmol), stirred for 10 minutes, and then for 16.5 hours after the addition of 0.3gm of 18-crown-6 and and 1.7gm (13mmol) of methyl 2-bromo-5-chloropentanoate. The solution was diluted with ethyl acetate and water. The organic phase was washed with water, washed with saturated sodium chloride, dried over sodium sulfate, and evaporated *in vacuo*. The residue was chromatograhed on silica gel eluting with a gradient hexane/10-25% ethyl ether to give product, 1.7gm, 40%, as an oil. $^1H$ NMR ($DMSOd_6$) δ: 1.35 (m, 1H), 1.60 (m, 1H), 2.10 (m, 1H), 2.20 (m, 1H), 3.55 (t, 2H), 3.60 (s, 3H), 3.80 (s, 3H), 4.00 (s, 2H), 6.60 (d, 1H), 6.85 (t, 1H), 7.00 (d, 1H), 7.10 (m, 1H), 7.15 (s, 1H), 7.20 (m, 4H).

C. Preparation of 4-methoxy-6-methoxycarbonyl-10-phenylmethyl-6,7,8,9-tetrahydropyrido[1,2-a]indole.

A solution of 1.8gm (4.7mmol) of the product from Part B and 4mL (15mmol) of tri-n-butyltin hydride in 50mL of toluene was heated to reflux and treated dropwise with a solution of 85mg (0.5mmol) of 2,2'-azobis(2-methylpropionitrile). The solution was refluxed 1 hour after the addition, cooled, evaporated *in vacuo*, taken up in ethyl acetate, shaken with aqueous potassium floride, and filtered. The organic phase was washed with saturated sodium chloride, dried over sodium sulfate, and evaporated *in vacuo* to give a mixture of 4-methoxy-6-methoxycarbonyl-10-phenylmethyl-6,7,8,9,9a,10-hexahydropyrido[1,2-a]indole and methyl 2-[3-phenylmethyl-7-methoxyindol-1-yl]pentanoate which was dissolved in 25mL of dioxane and stirred with 450mg (2mmol) of dichlorodicyanoquinone for 30 minutes. The solution was evaporated *in vacuo*, taken up in dichloromethane, filtered through florisil, and evaporated *in vacuo.* The residue was chromatographed on silica gel eluting with a gradient hexane/10-20% ethyl ether to give the title compound, 75mg, 5%, as an amorphous solid. $^1H$ NMR ($CDCl_3$) δ: 1.70 (m, 1H), 1.85 (m, 1H), 2.20 (m, 1H), 2.35 (m, 1H), 2.70 (m, 1H), 3.00 (m, 1H), 3.70 (s, 3H), 3.80 (s, 3H), 4.00 (q, 2H), 5.65 (m, 1H), 6.50 (d, 1H), 6.90 (t, 1H), 7.00 (d, 1H), 7.10 (m, 1H), 7.20 (m, 4H).

Example 6

Preparation of (4-carboxamido-9-phenylmethyl-4,5-dihydrothiopyrano[3,4-b]indol-5-yl)oxyacetic acid

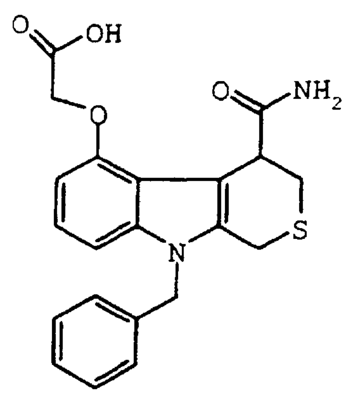

A. Preparation of methyl 3-(4-methoxyindol-3-yl)lactate.

To a solution of 4-methoxyindole (200mg, 1.36mmol) and methyl 2,3-epoxypropionate (258mg, 2.22mmol) in 40ml of carbon tetrachloride was added stannic chloride ( 0.16ml, 1.39mmol) dropwise at -5 to -10°C. The reaction mixture was stirred at that temperature for 1 hour and warmed up to room temperature slowly and with continous stirring. The reaction mixture was diluted with ethyl acetate and sodium bicarbonate solution, washed with brine, dried over sodium sulfate, and evaporated *in vacuo* to give 210mg a yellow oil which was subjected to flash column chromatography (2:1 to 1:1 hexanes:ethyl acetate) to give product, 157mg, 44%, as a yellow foam. $^1$H NMR ($CDCl_3$) d: 1.20 (t, 3H), 3.15(dd, 1H), 3.49 (dd, 1H), 3.95(s, 3H), 4.12 (q, 2H), 4.49(dd, 1H), 5.27 (s, 2H), 6.50(d, 1H), 6.83 (d, 1H), 7.08 (m, 2H), 7.31(m, 5H).

B. Preparation of a mixture of methyl 2-bromo-3-(4-methoxyindol-3-yl)propionate and methyl 2-bromomethyl-3-(4-methoxyindol-3-yl)acetate.

To a solution of the product from Part A (29mg, 0.11mmol) and triphenylphosphine (57.7mg, 0.22mmol) in 2ml of 1,2-dichloroethane was added a solution of 1,2-dibromotetrachloroethane (71.6mg, 0.22mmol) in lmL of 1,2-dichloroethane at -10°C. The reaction mixture was warmed up to room temperature and stirred for an additional 10-15minutes. It was then concentrated *in vacuo* and subjected to flash column chromatography (2:1 hexanes:ethyl ether) to give 31mg ,86%, of a mixture of methyl 2-bromo-3-(4-methoxyindol-3-yl)propionate and methyl 2-bromomethyl-3-(4-methoxyindol-3-yl)acetate as a yellow oil. $^1$H NMR ($CDCl_3$) d: 1.20 (t, 3H), 3.15(dd, 1H), 3.49 (dd, 1H), 3.95 (s, 3H), 4.12 (q, 2H), 4.49(dd, 1H), 5.27 (s, 2H), 6.50(d, 1H), 6.83 (d, 1H), 7.08 (m, 2H), 7.31(m, 5H).

C. Preparation of a mixture of methyl 2-bromo-3-(1-phenylmethyl-4-methoxyindol-3-yl)propionate and methyl 2-bromomethyl-3-(1-phenylmethyl-4-methoxyindol-3-yl)acetate.

The product mixture from Part B was dissolved in 5 ml of acetonitrile and ~lequivalent of potassium carbonate was added. This was heated to reflux overnight to form methyl 2-[4-methoxyindol-3,3-yl]spirocyclopropane carboxylate. To this reaction mixture was added 2 equivalents of benzyl bromide and the mixture refluxed overnight. The mix was filtered and concentrated. The residue was purified by flash column chromatography (97:1 hexanes:ether) to give 29mg , 66%, of a ca. 1:9 mixture of methyl 2-bromo-3-(1-phenylmethyl-4-methoxyindol-3-yl)propionate and methyl 2-bromomethyl-3-(1-phenylmethyl-4-methoxyindol-3-yl)acetate. $^1$H NMR ($CDCl_3$) d: 1.28 (t, 3H), 3.82 (d, 2H), 3.96 (s, 3H), 4.26 (q, 2H), 4.81 (t, 1H), 5.25 (s, 2H), 6.53 (d, 1H), 6.89 (d, 1H , 7.02-7.18 (m, 7H).

D. Preparation of methyl 2-acetylthiomethyl-3-(1-phenylmethyl-4-methoxyindol-3-yl)acetate.

To a solution of the product mixture from Part C (2.87g, 7.0mmol) in 15ml of tetrahydrofuran and 40ml of dimethylformamide was added 18-crown -6 (0.31gm) and potassium thioacetate (12.2g, 0.11mol) and then stirred at 50°C for 2 hours. The mixture was diluted with ethyl acetate and brine. The organic phase was washed, dried and concentrated. The residue was purified by HPLC and afforded 1.8g , 64.2%, of product. $^1$H NMR ($CDCl_3$) d: 1.19 (t, 3H), 2.28 (s, 3H), 3.54 (dd, 2H), 3.91 (s, 3H), 4.52 (t, 1H), 5.22 (s, 2H), 6.53 (d, 1H), 6.82 (d, 1H), 7.00 (s, 1H), 7.11 (m, 3H), 7.28 (m, 3H).

E. Preparation of methyl 2-mercaptomethyl-3-(1-phenylmethyl-4-methoxyindol-3-yl)acetate.

To a solution of the product from Part D (0.84g, 2.0mmol) in ethanol (70ml) was added potassium carbonate (4.lg, 30mmol). The reaction mixture was stirred at room temperature for 1.5 hours. It was quenched with hydrochloric acid solution and extracted with ethyl acetate, dried, concentrated to give the product, 0.74gm, 98%. $^1$H NMR ($CDCl_3$) d: 1.21 (t, 3H), 1.55 (t, 1H), 3.03 (m, 2H), 3.91 (s, 1H), 4.19 (q, 2H), 4.50 (t, 1H), 5.22 (s, 3H), 6.48 (d, 1H), 6.83 (d, 1H), 6.98 (s, 1H), 7.10 (m, 3H), 7.27 (m, 3H).

F. Preparation of methyl 2-methoxymethylmercaptomethyl-3-(1-phenylmethyl-4-methoxyindol-3-yl)acetate.

To a solution of the product from Part E (0.71g, 1.92mmol) in tetrahydrofuran (45ml) was added a few mgs of 18-crown-6 and potassium hexamethyldisilazide (4.54ml, 0.5M in toluene) at -75°C. The solution was stirred at -75°C for 3 minutes and then iodomethyl methyl ether (0.28ml,, mmol) was added and stirred for 20 minutes at -75°C. The reaction mixture was poured into a mixture of ethyl acetate and brine. The organic layer was washed with brine, dried over sodium sulfate, and concentrated *in vacuo*. The residue was purified by column chloromatigraphy ( 3:1 hexanes:ethyl acetate) to give product, 650mg, 82%, as a light yellow oil. $^1$H NMR ($CDCl_3$) d: 1.23 (t, 3H), 3.14 (m, 2H), 3.35 (s, 3H), 3.91 (s, 3H), 4.22 (q, 2H), 4.65 (d, 1H), 4.66 (t, 1H), 4.75 (d, 1H), 5.22 (s,2H), 6.51 (d, 1H), 6.90 (d, 1H), 7.00 (s, 1H), 7.07 (m, 3H), 7.28 (m, 3H).

G. Preparation of 4-methoxycarbonyl-5-methoxy-9-phenylmethyl-4,5-dihydrothiopyrano[3,4-b]indole.

To a solution of the product from Part F (518 mg, 1.25mmol) in dichloromethane (10ml) was quickly added one spatula of zinc bromide. The mixture was stirred at room temperature for 4.5 hours. The mix was poured into ethyl

acetate and sodium bicarbonate solution. The organic layer was washed with brine, dried over sodium sulfate, and concentrated *in vacuo*. The residue was purified by column chromatography (3:1 hexanes: ethyl acetate) to give 269mg, 56.4%, of the product as a yellow oil. $^1$H NMR ($CDCl_3$) d: 1.22 (t, 3H), 3.20 (dd, 1H), 3.59 (d, 1H), 3.72 (d, 1H), 3.83 (s, 3H), 4.21 (m 3H), 4.53 (t, 1H), 5.18 (d, 1H), 5.24 (d, 1H), 6.43 (d, 1H), 6.82 (d, 1H), 6.98 (d, 1H), 7.09 (t, 1H), 7.22 (m 4H) .

H. Preparation of 4-carboxamido-5-methoxy-9-phenylmethyl-4,5-dihydrothiopyrano[3,4-b]indole.

To a solution of the product from Part G (120mg, 0.31mmol) in benzene (15ml) was added freshly prepared methylchloroaluminum amide (0.67M, 9.3ml). The mixture was stirred at 50°C overnight. It was cooled, added to 1N hydrochloric acid, and diluted with ethyl acetate and brine. The organic layer was washed with brine, dried over sodium sulfate, and concentrated. The residue was purified by column chloromatography( 3:1 hexanes: ethyl acetate to ethyl acetate to 1% methanol in dichloromethane) to give product, 49.3mg, 45%. MS FIA 353.4 (M+1)

| Elemental Analyses for $C_{20}H_{20}N_2O_2S$: | | | |
|---|---|---|---|
| Calculated | C 68.16; | H 5.72; | N 7.95 |
| Found | C 68.31; | H 5.83; | N 8.05 |

I. Preparation of ethyl [4-carboxamido-9-phenylmethyl-4,5-dihydrothiopyrano[3,4-b]indol-5-yl]oxyacetate.

To a solution of the product from Part H (210mg, 0.60mmol) in dichloromethane ( 30ml) was added boron tribromide (10ml, 1M in dichloromethane). The mixture was stirred for 0.5 hour. The reaction mixture was poured into ice-water, extracted with 1% methanol in dichloromethane, washed with brine, dried, and concentrated. The crude 4-carboxamido-5-hydroxy-9-phenylmethyl-4,5-dihydrothiopyrano[3,4-b]indole was dissolved in 13ml DMF and the resulting solution was treated with sodium hydride (50mg, 60% in mineral oil, 1.25mmol) for 5 minutes and then with ethyl bromoacetate (0.09ml, 1.2mmol.) for 1.5 hours. The reaction mixture was diluted with ethyl acetate and brine. The organic layer was washed, dried, and concentrated. The residue was purified by column chromatography (1%-2% methanol in dichloromethane) to give product 79mg, 31%, as a yellow foam. MS FIA 425.2 (M+1)

| Elemental Analyses for $C_{23}H_{24}N_2O_4S$: | | | |
|---|---|---|---|
| Calculated | C 65.07; | H 5.57; | N 6.47 |
| Found | C 65.88; | H 5.57; | N 6.47 |

J. Preparation of (4-carboxamido-9-phenylmethyl-4,5-dihydrothiopyrano[3,4-b]indol-5-yl)oxyacetic acid.

To a solution of the product from Part I (53.7 mg, 0.13mmol) in a mixture solvent (5ml, tetrahydrofuran: methanol: water, 3:1:1) was added lithium hydroxide (~2.5equiv). The solution was stirred overnight, acidified to PH ~2, and extracted with ethyl acetate. The organic solution was dried over sodium sulfate and evaporated in *vacuo* to give the title compound, 37mg, 74%, as a yellow solid. MS FIA 397.1 (M+1).

| Elemental Analyses for $C_{21}H_{20}N_2O_4S$: | | | |
|---|---|---|---|
| Calculated | C 63.62; | H 5.08; | N 7.07 |
| Found | C 63.83; | H 5.33; | N 6.87 |

Example 7

3,4-dihydro-4-carboxamidol-5-methoxy-9-phenylmethylpyrano[3,4-b]indole

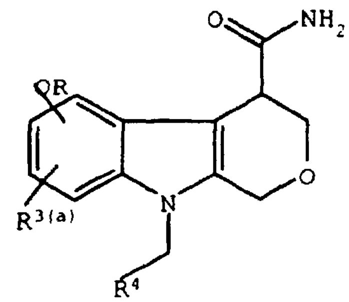

A. Preparation of ethyl [4-methoxyindol-3-yl]acetate.

To a solution of 2.94gm (20mmol) of 4-methoxyindole in 150ml of tetrahydrofuran was added slowly 13ml of n-butyl lithium (1.6M in hexane; 20mmol) followed by the slow addition of 20ml of zinc chloride (1.0M in ethyl ether; 20mmol) at 0-5°C. The cooling bath was removed and the solution stirred for 2 hours and then treated with 2.1ml (25mmol) of ethyl bromoacetate for 19 hours, diluted with ethyl acetate, washed with water, washed with brine, dried over sodium sulfate, and evaporated *in vacuo*. The residue was chromatographed on silica gel eluting with a gradient hexane/10-50% ethyl ether to give starting material (40%) and then product, 2.3gm, 50%, as an oil. $^1H$ NMR ($CDCl_3$) δ: 1,25 (t, 3H), 3.85 (s, 3H), 3.90 (s, 2H), 4.10 (q, 2H), 6.45 (d, 1H), 6.90 (d, 1H), 6.95 (s, 1H), 7.05 (t, 1H), 8.00 (br s, 1H).

B. Preparation of ethyl [4-methoxy-1-phenylmethylindol-3-yl]acetate.

A solution of 1.6gm (6.9mmol) of the product from Part A in 75ml of dimethylformamide and 10ml of tetrahydrofuran was treated in portions with 300mg of sodium hydride (60% in mineral oil; 7.5mmol) and then with 1.0ml (8.4mmol) of benzyl bromide for 4 hours, and then diluted with ethyl acetate and water. The organic phase was washed with water, washed with brine, dried over sodium sulfate, and evaporated *in vacuo*. The residue was chromatographed on silica gel eluting with a gradient hexane/10-20% ethyl ether to give product, 1.0gm, 45%, as an oil. $^1H$ NMR ($CDCl_3$) δ: 1.25 (t, 3H), 3.85 (s, 3H), 3.90 (s, 2H), 4.10 (q, 2H), 5.25 (s, 2H), 6.50 (d, 1H), 6.85 (d, 1H), 6.95 (s, 1H), 7.05 (t, 1H), 7.10 (d, 2H), 7.25 (m, 3H). MS ES+ 324.0 (M+1).

C. Preparation of ethyl 2-[4-methoxy-1-phenylmethylindol-3-yl]-3-phenylmethoxypropionate.

To a stirred solution of the product from Part B (1.4g, 4.3mmol) in 50mL of tetrahydrofuran was added potassium hexamethydisilazide (9.54 mL, 0.5M in toluene; 4.77mmol) slowly at -75°C under nitrogen. The resulting reaction mixture was stirred for a couple minutes and treated with chloromethyl benzyl ether (1.7g, 8.6mmol) at -75°C. The reaction mixture was stirred at -75°C for 0.5 hour and poured into a mixture of brine and ethyl acetate. The organic layer was washed with brine, dried and concentrated *in vacuo.* The residue was purified by flash column chromatography (3:1 hexanes: ethyl acetate) to give product as a yellow oil, 1.34g, 70.3%. $^1H$ NMR ($CDCl_3$) δ : 1.22 (t, 3H), 3.88 (s, 3H), 3.94 (dd, 1H), 4.21 (m, 3H), 4.56 (s, 2H), 4.75 (dd, 1H), 5.20 (s, 2H), 6.40 (d, 1H), 6.81 (d, 1H), 7.02-7.34 (m, 7H).

D. Preparation of ethyl 2-[4-methoxy-1-phenylmethylindol-3-yl]-3-hydroxypropionate.

To a stirred solution of the product from Part C (0.33g) in ethyl acetate (50mL) was added 5% Pd/C (0.17g) and 1mL of 1N hydrochloric acid. The reaction mixture was stirred under *ca.* 1 atmosphere of hydrogen at room temperature overnight. The reaction mixture was filtered, neutralized with sodium bicarbonate solution, and washed with brine. The organic layer was dried over sodium sulfate and concentrated *in vacuo* to give product, 0.23g, 89%,as a yellow oil. $^1H$ NMR ($CDCl_3$) δ : 1.21(t, 3H), 3.87 (s, 3H), 3.92 (dd, 1H), 4.20 (m, 3H), 4.44 (dd, 1H), 5.21 (s, 2H), 6.43 (d, 1H), 6.84 (d, 1H), 6.98 (s, 1H), 7.00 (m, 3H), 7.30 (m, 3H).

E. Preparation of ethyl 2-[4-methoxy-1-phenylmethylindol-3-yl]-3-methoxypropionate.

To a stirred solution of the product from Part D (0.26g, 0.74mmol) in 18mL of tetrahydrofuran was added potassium hexamethyldisilazide (1.63 mL, 0.5M in toluene, 0.815mmol) slowly at -75°C. To the reaction mixture was added iodomethyl methyl ether (0.13mL, 1.48mmol) at -75°C after 2 minutes stirring at the same temperature. The mixture was diluted with brine and ethyl acetate after 15 minutes at -75°C. The organic layer was washed with brine, dried over sodium sulfate, and concentrated *in vacuo*. The residue was purified by column chromatography (4:1 to 3: 1 hexanes:ethyl acetate) to give product, 0.23g, 79.3%, as a yellow oil. $^1H$ NMR ($CDCl_3$) δ : 1.21 (t, 3H), 3.35 (s, 3H), 3.91 (s, 3H), 9.95 (m, 2H), 4.22 (q, 2H), 4.65 (s, 2H), 4.72 (dd, 1H), 5.21 (s, 2H), 6.41 (d, 1H), 6.82 (d, 1H), 7.04 (m, 4H), 7.24 (m, 3H).

F. Preparation of 3,4-dihydro-4-ethoxycarbonyl-5-methoxy-9-phenylmethylpyrano[3,4-b]indole.

To a stirred solution of boron trifluoride etherate (0.071mL, 0.55mmol) in dichloromethane (6mL) was added a solution of the product from Part E (148 mg, 0.37mmol) in dichloromethane (4mL) at 0-5°C slowly. The reaction mixture was warmed up to room temperature and stirred for 0.5 hour to complete the reaction. The reaction mixture was diluted with ethyl acetate and brine. The organic layer was washed with brine, dried over sodium sulfate, and concentrated *in vacuo*. The residue was chromatographed on silica gel (1:1 hexanes:ethyl ether) to give product, 49.3mg, 36.2%, as a white solid. $^1H$ NMR ($CDCl_3$) δ : 1.21 (t, 3H), 3.88(s, 3H), 4.05 (dd, 1H), 4.15 (m, 1H), 4.24 (m, 3H), 4.60 (d, 1H), 4.78 (d, 1H), 5.04 (d, 1H), 5.18 (d, 1H), 6.44 (d, 1H), 6.82 (d, 1H), 7.01 (m, 3H), 7.22 (m, 3H).

G. Preparation of 3,4-dihydro-4-carboxamidol-5-methoxy-9-phenylmethylpyrano[3,4-b]indole.

To a solution of the product from Part F (490mg, 1.34 mmol) in benzene (60-80mL) was added freshly prepared methylchloroaluminum amide (0.67M, 60ml, 40mmol). The reaction mixture was stirred at 50°C for 24 hours, cooled, decomposed by the addition of 1N hydrochloric acid, and diluted with ethyl acetate and brine. The organic layer was washed with brine, dried over sodium sulfate, and concentrated. The residue was purified by column chloromatography on silica gel eluting with a gradient dichloromethane/ 1-2% methanol to give product, 335mg, 74.6%. MS FIA 337.2 (M+1)

| Analyses for $C_{20}H_{20}N_2O_3$: | | | |
|---|---|---|---|
| Calculated | C, 71.41; | H, 5.99; | N, 8.33 |
| Found | C, 71.51; | H, 6.19; | N, 8.26 |

Example 8

Preparation of 2-[(9 -benzyl-4-carbamoyl-1,2,3,4-tetrahydro-beta-carbolin-5-yl)oxy]acetic acid

A. Preparation of 4-(tertbutyldimethylsilyl)oxyindole.

Imidazole (15.3 g, 225 mmol) was added to a solution of 4-hydroxyindole (20 g, 150 mmol) in 300 mL of anhydrous methylene chloride at ambient temperature. The resulting mixture was treated with tert-butyldimethylsilyl chloride (25 g, 165 mmol). After stirring overnight at ambient temperature, the reaction mixture was poured into 300 mL of water. The layers were separated, and the aqueous phase was extracted with methylene (2 X 100 mL). The combined organic layers were dried over sodium sulfate, filtered and concentrated *in vacuo* to a black oil. The crude residue was purified on a Prep 500 (silica gel; 0% to 5% ethyl acetate/hexanes) to give the title compound as a light purple waxy solid in quantitative yield.
MS (ion spray, $NH_4OAc$) m/e $[M+1]^+$ 248, $[M-1]^-$ 246.

| Elemental Analysis for $C_{14}H_{21}NOSi$ : | | | |
|---|---|---|---|
| Calculated | C 67.96; | H 8.55; | N 5.66 |
| Found | C 69.10; | H 8.79; | N 5.70 |

B. Preparation of Ethyl [4-(tert-H-butyldimethylsilyl)oxyindole]-3-acetic acid

A solution of indole (78) (247 mg, 1.00 mmol) in dry tetrahydrofuran (2 mL) under a nitrogen atmosphere was

cooled to -10°C then n-butyllithium (0.625 mL, 1.00 mmol), 1.6 M in hexanes, was added dropwise over 30 sec by syringe. The resultant solution was stirred 15 minutes zinc chloride (1.0 mL, 1.0 mmol), 1 M in ether, was added all at once. The solution was stirred 2 hours while warming to ambient temperature. To this solution was added ethyl iodoacetate (0.118 mL, 1.00 mmol) all at once. The reaction mixture darkened but remained clear. The mixture was stirred 3 hours at ambient temperature concentrated *in vacuo*. The residue was purified directly on silica gel (30 X 35 mm column) eluting with methylene chloride. Concentration of the appropriate fractions yielded 192 mg (57.8%) of the titled product as a white solid.
MS (ion spray, $NH_4OAc$) m/e $[M+1]^+$ 334, $[M-1]^-$ 332.

| Elemental Analyses for $C_{18}H_{27}NO_3Si$: | | | |
|---|---|---|---|
| Calculated | C 64.86; | H 8.11; | N 4.20 |
| Found | C 65.11; | H 8.02; | N 4.24 |

C. Preparation of Ethyl [2,9-bis-benzyl-5-(tert-butyldimethylsilyl)oxy-1,2,3,4-tetrahydro-beta-carboline]-4-acetic acid

A solution of the ester (79) (5.08 g, 15.2 mmol) in dry tetrahydrofuran (100 mL) was cooled to -78°C then treated dropwise with 0.5 M potassium bis(trimethylsilyl)amide in toluene (32 mL, 16 mmol). The resultant solution was stirred 10 min then benzyl iodide (3.32g, 15.2 mmol) was added all at once. The cooling bath was removed, the mixture warmed quickly to 0°C, then slowly to ambient temperature. After stirring 75 minutes at ambient temperature the mixture was concentrated *in vacuo*. The residue was taken up in ether and washed successively with 10% aqueous citric acid, water and saturated sodium bicarbonate solution. The ethereal solution was dried over magnesium sulfate and concentrated *in vacuo*. The residue was purified on silica gel (70 X 130 mm column) eluting with 500 mL 1:1 methylene chloride/hexanes then 500 mL methylene chloride. The appropriate fractions were combined and concentrated *in vacuo* to yield 5.90 g (91%) of ethyl [1-benzyl-4-(tert-butyldimethylsilyl)oxyindole]-3-acetic acid as a brown oil. Benzyl amine (2.14 g, 20.0 mmol) and paraformaldehyde (1.80 g, 120 mmol) were combined and warmed to reflux in anhydrous methanol (10 mL) for 2 hours. The mixture was concentrated *in vacuo* and dried under vacuum for 30 minutes to yield crude benzyl bis(methoxymethyl)amine as a water white oil. This material was used immediately without purification. To a cooled solution of ethyl [1-benzyl-4-(tert-butyldimethylsilyl)oxyindole]-3-acetic acid (190 mg, 0.45 mmol) in dry tetrahydrofuran (2 mL) was added potassium bis(trimethylsilyl)amide (0.98 mL, 0.49 mmol), 0.5 M in toluene, dropwise by syringe. After stirring the mixture 10 minutes, trimethylsilylchloride (0.057 mL, 0.45 mmol) was added all at once. The mixture was allowed to warm to ambient temperature then concentrated *in vacuo*. The residue was dried 30 minutes under vacuum to yield the trimethylsilylketene acetal (81). The residual ketene acetal (81) was immediately dissolved in methylene chloride (30 mL) to which was added freshly prepared benzyl bis(methoxymethyl)amine (175 mg, 0.90 mmol). The mixture was cooled to -78°C and treated with 1 M zinc chloride in ether(0.9 mL, 0.9 mmol). The mixture was allowed to warm to ambient temperature and stirred for an additional 45 minutes. The mixture was washed with saturated sodium bicarbonate solution then passed thourough a silica gel plug eluting with 1:4 ethyl acetate/hexane. The desired fractions were combined and concentrated *in vacuo* then further purified on an SCX cartridge (1g, Varian) with methanol and ammonia. The desired fractions were combined, concentrated and purified on silica gel eluting with methylene chloride to yield 34 mg (14%) of the titled tricyclic indole. MS (ion spray, $NH_4OAc$) m/e $[M+1]^+$ 555.

| Elemental Analyses for $C_{34}H_{42}N_2O_3Si$: | | | |
|---|---|---|---|
| Calculated | C 73.64; | H 7.58; | N 5.05 |
| Found | C 73.42; | H 7.61; | N 5.15 |

D. Preparation of ethyl 2-[(2,9-bis-benzyl-4-carbamoyl-1,2,3,4-tetrahydro-beta-carbolin-5-yl)oxy]acetic acid

A solution of 565 mg (1.02 mmol) of the compound of Part C in 10 mL 1:1 methanol/tetrahydrofuran was treated with 5 mL (5 mmol) 1 N lithium hydroxide under an atmosphere of nitrogen. The mixture was warmed briefly, allowed to stir at ambient temperature for 2 hours then concentrated *in vacuo* to about 5 mL. The pH of the solution was adjusted to ~5 to 6 with 1 N hydrochloric acid. The resultant precipitate was collected and dried to yield 430 mg (102%) of hydroxy acid. This product was suspended with hydroxybenzotriazole (160 mg, 1.19mmol) and 1-(3-dimethylaminopropyl) -3-ethylcarbodiimide hydrochloride (940 mg, 2.30 mmol) in 30 mL of 1:1 tetrahydrofuran/methylene chloride. The mixture was stirred vigorously for 10 minutes, saturated with ammonia gas, stirred vigorously for 1 hour, then concentrated *in vacuo*. The residue was partitioned between ethyl acetate and saturated sodium bicarbonate solution. The ethyl acetate solution was dried over anhydrous magnesium sulfate, filtered and concentrated *in vacuo*. The residue was passed thourough a plug of silica gel with ethyl acetate. The eluant was evaporated to yield 175 mg

(43%) of the carboxamide.

This compound was dissolved in 3 mL dry tetrahydrofuran, cooled to -70°C and treated with 0.5 M potassium bis(trimethylsilyl)amide in toluene (0.85 mL, 0.425 mmol). The solution was stirred 10 min then ethyl bromoacetate was added all at once. The reaction was stirred 6 hours while warming to ambient temperature. The mixture was concentrated *in vacuo* and the residue purified on silica gel eluting with ethyl acetate to yield 86 mg (41%) of the title compound. MS (ion spray, $NH_4OAc$) m/e $[M+1]^+$ 498.

| Elemental Analyses for $C_{30}H_{31}N_3O_4$: | | | |
|---|---|---|---|
| Calculated | C 72.43; | H 6.24; | N 8.45 |
| Found | C 72.54; | H 6.36; | N 8.64 |

E. Preparation of 2-[(2,9-bis-benzyl-4-carbamoyl-1,2,3,4-tetrahydro-beta-carbol-5-yl)oxy]acetic acid

A solution of the compound from Part D (78 mg, 0.16 mmol) in 2 mL 1:1 tetrahydrofuran/methanol was stirred with 1 M lithium hydroxide (0.63 mL, 0.63 mmol) for 3 hours. The mixture was concentrated *in vacuo* to give a white solid. The solid was suspended in 2 mL water and the pH adjusted to ~ 5 to 6 with 1 N hydrochloric acid forming a somewhat different white solid. The new solid was collected by filtration and dried under vacuum to yield 68 mg (93%) of the title compound. MS (ion spray, $NH_4OAc$) m/e $[M+1]^+$ 470.

| Elemental Analyses for $C_{28}H_{27}N_3O_4 \cdot 0.8\ H_2O$: | | | |
|---|---|---|---|
| Calculated | C 69.49; | H 5.96; | N 8.68 |
| Found | C 69.50; | H 5.64; | N 8.54 |

F. Preparation of 2-[(9-benzyl-4-carbamoyl-1,2,3,4-tetrahydro-beta-carbolin-5-yl)oxy]acetic acid hydrochloride

A suspension of the compound from part E (68 mg, 0.14 mmol) was treated with 3-4 drops of 1N HCl to effect solution. To the solution was added 10% palladium on carbon (70 mg). The flask was appropriately purged with nitrogen and hydrogen then stirred under a hydrogen atmosphere for 18 h. The mixture was filtered and the solids thoroughly washed with methanol. The filtrate was concentrated in vacuo to yield a mixture of acid and methyl ester. The mixture was treated with aqueous 1N LiOH (0.3 mL) in about 2 mL methanol over 2 h. The mixture was concentrated in vacuo and the residue acidified to pH=5 with 1 N HCl causing a precipitate to form. The precipitate was collected by filtration. The filtrate was concentrated in vacuo to leave a residue. The collected solid and the residue were purified by reverse phase chromatography to yield 31mg (68%) of the title compound as the HCl salt. MS (ion spray) m/e $[M+1]^+$ 380. IR (KBr, $cm^{-1}$) 3393(br), 3100-2500 (COOH), 1735, 1671, 1638, 1615, 1445, 1263, 1133, 731,722.

<u>Therapeutic Use of Tricyclic Compounds</u>

The compounds described herein are believed to achieve their beneficial therapeutic action principally by direct inhibition of human $sPLA_2$, and not by acting as antagonists for arachidonic acid, nor other active agents below arachidonic acid in the arachidonic acid cascade, such as 5-lipoxygenases, cyclooxygenases, etc.

The method of the invention for inhibiting $sPLA_2$ mediated release of fatty acids comprises contacting $sPLA_2$ with an therapeutically effective amount of the compound of Formula (I) or its salt.

The compounds of the invention may be used in a method of treating a mammal (e.g., a human) to alleviate the pathological effects of septic shock, adult respiratory distress syndrome, pancreatitus, trauma, bronchial asthma, allergic rhinitis, and rheumatoid arthritis; wherein the method comprises administering to the mammal a compound of formula (I) in a therapeutically effective amount. A "therapeutically effective" amount is an amount sufficient to inhibit $sPLA_2$ mediated release of fatty acid and to thereby inhibit or prevent the arachidonic acid cascade and its deleterious products. The therapeutic amount of compound of the invention needed to inhibit $sPLA_2$ may be readily determined by taking a sample of body fluid and assaying it for $sPLA_2$ content by conventional methods.

Throughout this document, the person or animal to be treated will be described as a "mammal", and it will be understood that the most preferred subject is a human. However it must be noted that the study of adverse conditions of the central nervous system in non-human animals is only now beginning, an that some instances of such treatments are coming into use. Accordingly, use of the present compounds in non-human animals is contemplated. It will be understood that the dosage ranges for other animals will necessarily be quite different from the doses administered to humans, and accordingly that the dosage ranges described be recalculated. For example, a small dog may be only $1/10^{th}$ of a typical human's size, and it will therefore be necessary for a much smaller dose to be used. The determination

of an effective amount for a certain non-human animal is carried out in the same manner described below in the case of humans, and veterinarians are well accustomed to such determinations.

Pharmaceutical Formulations of the Invention

As previously noted the compounds of this invention are useful for inhibiting $sPLA_2$ mediated release of fatty acids such as arachidonic acid. By the term, "inhibiting" is meant the prevention or therapeutically significant reduction in release of $sPLA_2$ initiated fatty acids by the compounds of the invention. By "pharmaceutically acceptable" it is meant the carrier, diluent or excipient must be compatible with the other ingredients of the formulation and not deleterious to the recipient thereof.

In general, the compounds of the invention are most desirably administered at a dose that will generally afford effective results without causing any serious side effects and can be administered either as a single unit dose, or if desired, the dosage may be divided into convenient subunits administered at suitable times throughout the day.

The specific dose of a compound administered according to this invention to obtain therapeutic or prophylactic effects will, of course, be determined by the particular circumstances surrounding the case, including, for example, the route of administration, the age, weight and response of the individual patient, the condition being treated and the severity of the patient's symptoms. Typical daily doses will contain a non-toxic dosage level of from about 0.01 mg/kg to about 50 mg/kg of body weight of an active compound of this invention.

Preferably the pharmaceutical formulation is in unit dosage form. The unit dosage form can be a capsule or tablet itself, or the appropriate number of any of these. The quantity of active ingredient in a unit dose of composition may be varied or adjusted from about 0.1 to about 1000 milligrams or more according to the particular treatment involved. It may be appreciated that it may be necessary to make routine variations to the dosage depending on the age and condition of the patient. The dosage will also depend on the route of administration.

A "chronic" condition means a deteriorating condition of slow progress and long continuance. As such, it is treated when it is diagnosed and continued throughout the course of the disease. An "acute" condition is an exacerbation of short course followed by a period of remission. In an acute event, compound is administered at the onset of symptoms and discontinued when the symptoms disappear.

Pancreatitis, trauma-induced shock, bronchial asthma, allergic rhinitis and rheumatoid arthritis may occur as an acute event or a chronic event. Thus, the treatment of these conditions contemplates both acute and chronic forms. Septic shock and adult respiratory distress, on the other hand, are acute conditions treated when diagnosed.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the compounds of the invention together with a pharmaceutically acceptable carrier or diluent therefor. The present pharmaceutical formulations are prepared by known procedures using well known and readily available ingredients.

In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. Solid form formulations include powders, tablets and capsules. A solid carrier can be one or more substances which may also act as flavoring agents, lubricants, solubilisers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc.

In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from about 1 to about 99 weight percent of the active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The active ingredient can be dissolved or suspended in a pharmaceutically acceptable carrier, such as sterile water, sterile organic solvent or a mixture of both. The active ingredient can often be dissolved in a suitable organic

solvent, for instance aqueous propylene glycol. Other compositions can be made by dispersing the finely divided active ingredient in aqueous starch or sodium carboxymethyl cellulose solution or in a suitable oil. "Active ingredient", refers to a compound according to Formula (I) or a pharmaceutically acceptable salt, or solvate thereof.

Formulation 1

Hard gelatin capsules are prepared using the following ingredients:

| | Quantity (mg/capsule) |
|---|---|
| 2-[4-oxo-5-carboxamido-9-(4-methylbenzyl)-9*H*-pyrido[3,4-*b*]indolyl]acetic acid | 250 |
| Starch, dried | 200 |
| Magnesium stearate | 10 |
| Total | 460 mg |

Formulation 2

A tablet is prepared using the ingredients below:

| | Quantity (mg/tablet) |
|---|---|
| 2-[4-oxo-5-carboxamido-9-[4-(trifluoromethyl)benzyl]-9*H*-pyrido[3,4-*b*]indolyl]acetic acid | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg

Formulation 3

An aerosol solution is prepared containing the following components:

| | Weight |
|---|---|
| 2-[4-oxo-5-carboxamido-9-(3-benzoylbenzyl)-9*H*-pyrido[3,4-*b*]indolyl]acetic acid | 0.25 |
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

| | |
|---|---|
| 2-[4-oxo-5-carboxamido-9-(2,4,6-trifluorobenzyl)-9*H* -pyrido[3,4-*b* ]indolyl]acetic acid | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinylpyrrolidone is mixed with the resultant powder, and the mixture then is passed through *a* No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| 2-[4-oxo-5-carboxamido-9-(2-fluorobenzyl)-9*H*-pyrido[3,4-*b*]indolyl]acetic acid tetrahydrocarbazole-4-carboxamide | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| 2-[4-oxo-5-carboxamido-9-pentafluorobenzyl-9*H*-pyrido[3,4-*b*]indolyl]acetic acid | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions, each containing 50 mg of active ingredient per 5 ml dose, are made as follows:

| | |
|---|---|
| 2-[4-oxo-5-carboxamido-9-(3,4,5-trimethoxybenzyl)-9*H*-pyrido[3,4-*b*]indolyl]acetic acid | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| 2-[4-oxo-5-carboxamido-9-(3,5-difluorobenzyl)-9*H*-pyrido[3,4-*b*]indolyl]acetic acid | 100 mg |
| Isotonic saline | 1,000 ml |

The solution of the above ingredients generally is administered intravenously to a subject at a rate of 1 ml per minute.

Assay Experiments

Assay Example 1

The following chromogenic assay procedure was used to identify and evaluate inhibitors of recombinant human secreted phospholipase $A_2$. The assay described herein has been adapted for high volume screening using 96 well microtiter plates. A general description of this assay method is found in the article, "Analysis of Human Synovial Fluid Phospholipase $A_2$ on Short Chain Phosphatidylcholine-Mixed Micelles: Development of a Spectrophotometric Assay Suitable for a Microtiterplate Reader", by Laure J. Reynolds, Lori L. Hughes, and Edward A Dennis, Analytical Biochemistry, 204, pp. 190-197, 1992 (the disclosure of which is incorporated herein by reference) :

Reagents:

REACTION BUFFER -

$CaCl_2.2H_2O$ (1.47 g/L)
KCl (7.455 g/L)
Bovine Serum Albumin (fatty acid free) (1 g/L) (Sigma A-7030, product of Sigma Chemical Co. St. Louis MO, USA)
TRIS HCl (3.94 g/L)
pH 7.5 (adjust with NaOH)

ENZYME BUFFER -

0.05 $NaOAc.3H_2O$, pH 4.5
0.2 NaCl
Adjust pH to 4.5 with acetic acid

DTNB - 5,5'-dithiobis-2-nitrobenzoic acid
RACEMIC DIHEPTANOYL THIO - PC

racemic 1,2-bis(heptanoylthio)-1,2-dideoxy-sn-glycero-3-phosphorylcholine
TRITON X-100™ prepare at 6.249 mg/ml in reaction buffer to equal 10uM
TRITON X-100™ is a polyoxy ethylene non-ionic detergent supplied by
Pierce Chemical Company,
3747 N. Meridian Road, Rockford, Illinois
61101.

REACTION MIXTURE -

A measured volume of racemic dipheptanoyl thio PC supplied in chloroform at a concentration of 100 mg/ml is taken to dryness and redissolved in 10 millimolar TRITON X-100™ nonionic detergent aqueous solution. Reaction Buffer is added to the solution, then DTNB to give the Reaction Mixture.

The reaction mixture thus obtained contains 1mM diheptanoly thio-PC substrate, 0.29 mm Triton X-100™ detergent, and 0.12 mm DTMB in a buffered aqueous solution at pH 7.5.

Assay Procedure:

1. Add 0.2 ml reaction mixture to all wells;
2. Add 10 ul test compound (or solvent blank) to appropriate wells, mix 20 seconds;
3. Add 50 nanograms of $sPLA_2$ (10 microliters) to appropriate wells;
4. Incubate plate at 40°C for 30 minutes;
5. Read absorbance of wells at 405 nanometers with an automatic plate reader.

All compounds were tested in triplicate. Typically, compounds were tested at a final concentration of 5 ug/ml. Compounds were considered active when they exhibited 40% inhibition or greater compared to uninhibited control reactions when measured at 405 nanometers. Lack of color development at 405 nanometers evidenced inhibition. Compounds initially found to be active were reassayed to confirm their activity and, if sufficiently active, $IC_{50}$ values were determined. Typically, the $IC_{50}$ values were determined by diluting test compound serially two-fold such that the final concentration in the reaction ranged from 45 ug/mL to 0.35 ug/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of $IC_{50}$ values. $IC_{50}$ were determined by plotting log concentration versus inhibition values in the range from 10-90% inhibition.

Compounds of the instant invention (Examples 1-8) were tested in Assay Example 1 and were found to be effective at concentrations of less than 100μM.

Assay Example 2

Method:

Male Hartley strain guinea pigs (500-700g) were killed by cervical dislocation and their heart and lungs removed intact and placed in aerated (95% $O_2$:5% $CO_2$) Krebs buffer. Dorsal pleural strips (4x1x25mm) were dissected from intact parenchymal segments (8x4x25mm) cut parallel to the outer edge of the lower lung lobes. Two adjacent pleural strips, obtained from a single lobe and representing a single tissue sample, were tied at either end and independently attached to a metal support rod. One rod was attached to a Grass force-displacement transducer Model FTO3C, product of Grass Medical Instruments Co., Quincy, MA, USA). Changes in isometric tension were displayed on a monitor and thermal recorder (product of Modular Instruments, Malvern, PA). All tissues were placed in 10 ml jacketed tissue baths maintained at 37°C. The tissue baths were continuously aerated and contained a modified Krebs solution of the following composition (millimolar) NaCl, 118.2; KCl, 4.6; $CaCl_2 \cdot 2H_2O$, 2.5; $MgSO_4 \cdot 7H_2O$, 1.2; $NaHCO_3$, 24.8; $KH_2PO_4$, 1.0; and dextrose, 10.0. Pleural strips from the opposite lobes of the lung were used for paired experiments. Preliminary data generated from tension/response curves demonstrated that resting tension of 800mg was optimal. The tissues were allowed to equilibrate for 45 min. as the bath fluid was changed periodically.

Cumulative concentration-response curves:

Initially tissues were challenged 3 times with KCl (40 mM) to test tissue viability and to obtain a consistent response. After recording the maximal response to KCl, the tissues were washed and allowed to return to baseline before the next challenge. Cumulative concentration-response curves were obtained from pleural strips by increasing the agonist concentration ($sPLA_2$) in the tissue bath by half-$log_{10}$ increments while the previous concentration remained in contact with the tissues (Ref.1, supra.). Agonist concentration was increased after reaching the plateau of the contraction elicited by the preceding concentration. One concentration-response curve was obtained from each tissue. To minimize variability between tissues obtained from different animals, contractile responses were expressed as a percentage of the maximal response obtained with the final KCl challenge. When studying the effects of various drugs on the contractile effects of $sPLA_2$, the compounds and their respective vehicles were added to the tissues 30 minutes prior to starting the $sPLA_2$ concentration-response curves.

Statistical analysis:

Data from different experiments were pooled and presented as a percentage of the maximal KCl responses (mean ± S.E.). To estimate the drug induced rightward shifts in the concentration response curves, the curves were analyzed simultaneously using statistical nonlinear modeling methods similar to those described by Waud (1976), Equation 26, p. 163, (Ref.2). The model includes four parameters: the maximum tissue response which was assumed the same for each curve, the $ED_{50}$ for the control curve, the steepness of the curves, and the $pA_2$, the concentration of antagonist that requires a two-fold increase in agonist to achieve an equivalent response. The Schild slope was determined to be 1, using statistical nonlinear modeling methods similar to those described by Waud (1976), Equation 27, p. 164 (Ref. 2). The Schild slope equal to 1 indicates the model is consistent with the assumptions of a competitive antagonist; therefore, the pA2 may be interpreted as the apparent $K_{B,}$ the dissociation constant of the inhibitor.

To estimate the drug-induced suppression of the maximal responses, $sPLA_2$ responses (10 ug/ml) were determined in the absence and presence of drug, and percent suppression was calculated for each pair of tissues. Representative examples of inhibitory activities are presented in Table 2, below.

Ref. 1 - Van, J.M.: Cumulative dose-response curves. II. Technique for the making of dose-response curves

in isolated organs and the evaluation of drug parameters. Arch. Int. Pharmacodyn. Ther., 143: 299-330, 1963.

Ref. 2 - Waud, D.: Analysis of dose-response relationships. in Advances in General and Cellular Pharmacology eds Narahashi, Bianchi 1:145-178, 1976.

Compounds of the instant invention (Examples 1-19) were tested in Assay Example 2 and were found to be effective at concentrations below 20μM.

Assay Example 3

$sPLA_2$ Transgenic Mice Assay

Materials & Methods

The mice utilized in these studies were mature, 6-8 month old, $ZnSO_4$-stimulated, hemizygous line 2608[a] transgenic mice (Fox et. al. 1996). Transgenic mice from this line express human $sPLA_2$ in the liver and other tissues and typically achieve levels of human $sPLA_2$ in their circulation of approximately 173 ± 10 ng/ml when maximally stimulated with $ZnSO_4$ (Fox, *et al*. 1996). *The* mice were housed under constant humidity and temperature and received food and water ad libitum. Animal room lighting was maintained on a 12-hour light/dark cycle and all experiments were performed at the same time of the day during the early morning light period.

For intravenous testing, compounds or vehicle were administered as an IV bolus via the tail vein in a volume of 0.15 ml. Vehicle consisted of 1-5% dimethylsulfoxide, 1-5% ethanol and 10-30% polyethylene glycol 300 in $H_2O$; the concentrations of these ingredients were adjusted according to the solubility of the compound. Mice were bled retro-orbitally prior to drug or vehicle administration and 30 minutes, 2 and 4 hours thereafter. Three to six mice were used for each dose. $PLA_2$ catalytic activity in the serum was assayed with a modified phosphatidylcholine/deoxycholine mixed micelle assay (Fox, *et al*. 1996, Schadlich, *et al*., 1987) utilizing 3 mM sodium deoxycholate and 1 mM 1-palmitoyl-2-oleoyl-sn-glycero-3-phosphocholine.

For oral testing, compounds were dissolved in 1-5% ethanol/10-30% polyethylene glycol 300 in $H_2O$ or were suspended in 5% dextrose in $H_2O$ and administered by oral gavage. Serum was prepared from retro-orbital blood and assayed for $PLA_2$ catalytic activity as above.

References

Fox, N., M. Song, J. Schrementi, J. D. Sharp, D. L. White, D. W. Snyder, L. W. Hartley, D. G. Carlson, N. J. Bach, R. D. Dillard, S. E. Draheim, J. L. Bobbitt, L. Fisher and E. D. Mihelich. 1996.
Eur. J. Pharmacol. 308: 195.
Schadlich, H.R., M. Buchler, and H. G. Beger, 1987, J. Clin. Chem. Clin.
Biochem. 25, 505.

Compounds of the instant invention were tested in Assay Example 3 and were found to be effective.

## Claims

1. A compound selected from the group consisting of

(*R,S*)-(9-benzyl-4-carbamoyl-1-oxo-3-thia-1,2,3,4-tetrahydrocarbazol-5-yl)oxyacetic acid;
(*R,S*)-(9-benzyl-4-carbamoyl-3-thia-1,2,3,4-tetrahydrocarbazol-5-yl)oxyacetic acid;
2-(4-oxo-5-carboxamido-9-benzyl-9*H*-pyrido[3,4-*b*]indolyl)acetic acid hydrochloride;
[N-benzyl-1-carbamoyl-1-aza-1,2,3,4-tetrahydrocarbazol-8-yl]oxyacetic acid;
4-methoxy-6-methoxycarbonyl-10-phenylmethyl-6,7,8,9-tetrahydropyrido[1,2-a]indole;
(4-carboxamido-9-phenylmethyl-4,5-dihydrothiopyrano[3,4-b]indol-5-yl)oxyacetic acid;
3,4-dihydro-4-carboxamidol-5-methoxy-9-phenylmethylpyrano[3,4-b]indole;
2-[(9 -benzyl-4-carbamoyl-1,2,3,4-tetrahydro-beta-carbolin-5-yl)oxy]acetic acid,
or a pharmaceutically acceptable racemate, solvate, tautomer, optical isomer, or salt thereof.

**2.** A pharmaceutical formulation comprising a compound as claimed in Claim 1 together with a pharmaceutically acceptable carrier or diluent therefor.

**3.** A pharmaceutical formulation adapted for the treatment of a condition associated with inhibiting $sPLA_2$, containing a compound as claimed in Claim 1 together with a pharmaceutically acceptable carrier or diluent therefor.

**4.** The use of a compound as claimed in Claim 1 for the manufacture of a medicament for selectively inhibiting $sPLA_2$ in a mammal.

**5.** The use of Claim 4 wherein the mammal is a human.

**6.** The use of a compound as claimed in Claim 1 for the manufacture of a medicament for treating rheumatoid arthritis, osteoarthritis, stroke, apoptosis, asthma, chronic bronchitis, acute bronchitis, cystic fibrosis, inflammatory bowel disease, or pancreatitis.

**7.** The use of a compound as defined in Claim 1 for the manufacture of a medicament for alleviating the pathological effects of sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis;
and related diseases.

## Patentansprüche

**1.** Verbindung, ausgewählt aus der Gruppe, die besteht aus

(R,S)-(9-Benzyl-4-carbamoyl-1-oxo-3-thia-1,2,3,4-tetrahydrocarbazol-5-yl)oxyessigsäure,
(R,S)-(9-Benzyl-4-carbamoyl-3-thia-1,2,3,4-tetrahydrocarbazol-5-yl)oxyessigsäure,
2-(4-Oxo-5-carboxamido-9-benzyl-9H-pyrido[3,4-b]indolyl)essigsäurehydrochlorid,
[N-Benzyl-1-carbamoyl-1-aza-1,2,3,4-tetrahydrocarbazol-8-yl]oxyessigsäure,
4-Methoxy-6-methoxycarbonyl-10-phenylmethyl-6,7,8,9-tetrahydropyrido[1,2-a]indol,
(4-Carboxamido-9-phenylmethyl-4,5-dihydrothiopyrano[3,4-b]indol-5-yl)oxyessigsäure,
3,4-Dihydro-4-carboxamido-5-methoxy-9-phenylmethylpyrano[3,4-b] indol,
2-[(9-Benzyl-4-carbamoyl-1,2,3,4-tetrahydro-β-carbolin-5-yl)oxy]essigsäure,

oder ein phannazeutisch annehmbares Razemat, Solvat, Tautomer, optisches Isomer oder Salz hiervon.

**2.** Pharmazeutische Formulierung, die eine Verbindung nach Anspruch 1 zusammen mit einem pharmazeutisch annehmbaren Träger oder Verdünnungsmittel hierfür enthält.

**3.** Pharmazeutische Formulierung, die zur Behandlung eines Zustands angepaßt ist, der mit der Hemmung der $sPLA_2$ zusammenhängt, die eine Verbindung nach Anspruch 1 zusammen mit einem pharmazeutisch annehmbaren Träger oder Verdünnungsmittel hierfür enthält.

**4.** Verwendung einer Verbindung nach Anspruch 1 zur Herstellung eines Arzneimittels zur selektiven Hemmung der $sPLA_2$ bei einem Säuger.

**5.** Verwendung nach Anspruch 4, worin der Säuger ein Mensch ist.

**6.** Verwendung einer Verbindung nach Anspruch 1 zur Herstellung eines Arzneimittels zur Behandlung von rheumatoider Arthritis, Osteoarthritis, Schlaganfall, Apoptose, Asthma, chronischer Bronchitis, akuter Bronchitis, cytischer Fibrose, entzündlicher Darmerkrankung oder Pankreatitis.

**7.** Verwendung einer Verbindung nach Anspruch 1 zur Herstellung eines Arzneimittels zur Linderung der pathologischen Effekte von Sepsis, septischem Schock, Atemstreßsyndrom beim Erwachsenen, Pankreatitis, Trauma induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, alkylosierender Spondylitis, Reiter Syndrom, psoriatischer Arthropathie, enterapathischer Spondylitis, juveniler Arthropathie oder juveniler alkylosierender Spondylitis, reaktiver Arthropathie, infektiöser oder postinfektiöser Artluitis, Gonokokkenarthritis, Tuberkulosearthritis, viraler Arthritis, pilzbedingter Arthritis, Syphilis-bedingter Arthritis, Lyme-Erkankung, Arthritis, die mit "vaskulitischen Syndromen" assoziiert ist, Polyarteritis nodosa, hyperempfindlicher Vaskulitis, Luegenec Granulomatose, Polymyalgia rheumatica, Gelenkszellarteritis, Arthropathie durch Calciumkristallablagerung, Pseudogicht, nicht-artikulärem Rheuma, Bursitis, Tenosynovitis, Epicondylitis (Tennisellenbogen), Carpaltunnelsyndrom, Verletzung durch wiederkehrende Tätigkeit (Tippen), verschiedenen Formen der Arthritis, neuropatischer Gelenkserkrankung (Charcotgelenk und Gelenke), Hämarthrose (hämarthrotisch), Purpura Schönlein-Hennoch, hypertropher Osteoarthropathie, multizentrischer Reticulohistiocytose, Arthritis, die mit bestimmten Erkrankungen assoziiert ist, Surcoilose, Hämochromatose, Sichelzellerkrankung und anderen Hämoglobinopathien, Hyperlipoproteinämie, Hypogammaglobulinämie, Hyperparathyreoidismus, Akromegalie, familiäres mediterranes Fieber, Behat Erkrankung, systemischer Lupus erythrematosis oder Polychondritisrückfall und verwandten Erkrankungen.

## Revendications

**1.** Composé choisi dans le groupe constitué de

l'acide (R,S)-(9-benzyl-4-carbamoyl-1-oxo-3-thia-1,2,3,4-tétrahydrocarbazol-5-yl)oxyacétique,
l'acide (R,S)-(9-benzyl-4-carbamoyl-3-thia-1,2,3,4-tétrahydrocarbazol-5-yl)oxy-acétique,
le chlorhydrate de l'acide 2-(4-oxo-5-carboxamido-9-benzyl-9H-pyrido[3,4-b]-indolyl)acétique,
l'acide [N-benzyl-1-carbamoyl-1-aza-1,2,3,4-tétrahydrocarbazol-8-yl]oxy-acétique,
le 4-méthoxy-6-méthoxycarbonyl-10-phénylméthyl-6,7,8,9-tétrahydropyrido[1,2-a]indole;
l'acide (4-carboxamido-9-phénylméthyl-4,5-dihydrothiopyrano[3,4-b]indol-5-yl)-oxyacétique,
le 3,4-dihydro-4-carboxamidol-5-méthoxy-9-phénylméthylpyrano[3,4-b]indole;
l'acide 2-[(9-benzyl-4-carbamoyl-1,2,3,4-tétrahydro-bêtacarbolin-5-yl)oxy]-acétique,

ou un racémate, un solvate, un tautomère, un isomère optique ou un sel pharmaceutiquement acceptable de celui-ci.

**2.** Formulation pharmaceutique comprenant un composé tel que revendiqué dans la revendication 1 avec un support ou un diluant pharmaceutiquement acceptable pour celui-ci.

**3.** Formulation pharmaceutique adaptée pour le traitement d'un état associé à l'inhibition de la $sPLA_2$, contenant un composé tel que revendiqué dans la revendication 1 avec un support ou un diluant pharmaceutiquement acceptable pour celui-ci.

**4.** Utilisation d'un composé tel que revendiqué dans la revendication 1 pour la préparation d'un médicament destiné à inhiber sélectivement la $sPLA_2$ chez un mammifère.

**5.** Utilisation de la revendication 4 dans laquelle le mammifère est un être humain.

**6.** Utilisation d'un composé tel que revendiqué dans la revendication 1 pour la préparation d'un médicament pour traiter l'arthrite rhumatoïde, l'ostéoarthrite, une attaque, l'apoptose, l'asthme, la bronchite chronique, la fibrose cystique, la maladie de l'intestin inflammatoire ou la pancréatite.

**7.** Utilisation d'un composé tel que défini dans la revendication 1 pour la préparation d'un médicament pour soulager

les effets pathologiques d'une septicémie, d'un choc septique, du syndrome de détresse respiratoire adulte, de la pancréatite, du choc induit par un traumatisme, de l'asthme bronchique, de la rhinite allergique, de l'arthrite rhumatoïde, de la fibrose cystique, d'une attaque, de la bronchite aiguë, de la bronchite chronique, de la bronchiolyte aiguë, de la bronchiolyte chronique, de l'ostéoarthrite, de la goutte, de la spondylarthropathie, de la spondylite ankylosante, du syndrome de Reiter, de l'arthropathie psoriatique, de la spondylite entéropathique, de l'arthropathie juvénile ou de la spondylite ankylosante juvénile, de l'arthropathie réactive, de l'arthrite infectieuse ou post-infectieuse, de l'arthrite gonococcique, de l'arthrite tuberculeuse, de l'arthrite virale, de l'arthrite fongique, de l'arthrite syphilitique, de la maladie de Lyme, de l'arthrite associée à des syndromes vascularitiques, de la périartérite noueuse, de la vascularite d'hypersensibilité, de la granulomatose de Luegenec, de la pseudopolyarthrite rhizomélique, de l'artérite articulaire à cellules, de l'arthropathie par dépôt de cristaux de calcium, de la pseudo-goutte, du rhumatisme non articulaire, de la bursite, de la ténosynovite, de l'épicondylite (tennis elbow), du syndrome du canal carpien, des lésions dues à une utilisation répétitive (dactylographie), de différentes formes d'arthrite, de la maladie neuropathique des articulations (charco et articulation), de l'hémarthrose, du purpura de Henoch-Schonlein, de l'ostéoarthropathie hypertrophique, de la réticulohistiocytose multicentrique, de l'arthrite associée à certaines maladies, de la surcoilose, de l'hémochromatose, de la drépanocytose et d'autres hémoglobinopathies, de l'hyperlipoprotéinémie, de l'hypogammaglobulinémie, de l'hyperparathyroïdie, de l'acromégalie, de la brucellose, de la maladie de Behat, du lupus érythémateux systémique ou de la polychondrite chronique et des maladies associées.